(19)

**Europäisches Patentamt**
**European Patent Office**
**Office européen des brevets**

(11) **EP 2 686 069 B1**

(12) **EUROPEAN PATENT SPECIFICATION**

(45) Date of publication and mention
of the grant of the patent:
**26.08.2015 Bulletin 2015/35**

(51) Int Cl.:
***A61N 7/02*** *(2006.01)* ***A61B 17/225*** *(2006.01)*

(21) Application number: **12709749.1**

(22) Date of filing: **02.03.2012**

(86) International application number:
**PCT/US2012/027414**

(87) International publication number:
**WO 2012/125304 (20.09.2012 Gazette 2012/38)**

(54) **SYSTEM FOR NON-INVASIVE TRANSCRANIAL INSONATION**

SYSTEM ZUR NICHTINVASIVEN TRANSKRANIALEN DOPPLERUNTERSUCHUNG

SYSTÈME POUR UN TRAITEMENT AUX ULTRASONS TRANSCRANIEN NON INVASIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2011 US 201161453771 P
13.08.2011 US 201113209385**

(43) Date of publication of application:
**22.01.2014 Bulletin 2014/04**

(73) Proprietor: **Cerevast Therapeutics
Redmond, WA 98052 (US)**

(72) Inventors:
• **ALLEMAN, Anthony, John
Sherwood, OR 97140 (US)**

• **BARNARD, William
Maple Valley, WA 98038 (US)**
• **RADFORD, Randal, Lee
Burien, WA 98166 (US)**

(74) Representative: **Müller-Boré & Partner
Patentanwälte PartG mbB
Friedenheimer Brücke 21
80639 München (DE)**

(56) References cited:
**WO-A2-2007/044469     US-A1- 2005 085 748
US-A1- 2006 184 070     US-A1- 2007 167 765**

EP 2 686 069 B1

**Description**

TECHNICAL FIELD

The present invention relates to ultrasound devices and, in particular, to a portable ultrasound system having stereotactic features configured to ensure that a pre-defined portion of the cerebral vasculature is predictably insonated, once the system is positioned on the head of a subject, without conventionally-required feedback from the clinician.

BACKGROUND ART

Numerous devices for ultrasound irradiation of a head of a subject (referred herein, for simplicity, as "ultrasound devices" or "US devices") are known in the art. However, the use of most, if not all, of the currently available US devices imposes certain requirements on the insonation procedure that limits the efficiency and flexibility of operation of such devices. For example, because the purpose of such an US device is to interrogate a portion of cerebral vasculature, such devices must be carefully positioned and oriented, by an operator (or clinician), with respect to the subject's head to ensure that the ultrasound beams produced by the transducers of the US device properly directed towards the portion of cerebral vasculature in question. Such positioning is often facilitated with a preliminary sonographic imaging (for example, Doppler imaging) that lengthens the time of the overall procedure and produces an image of the system of the brain blood vessels in relations to the chosen spatial system of coordinates. It is in reliance on these Doppler images that the operator further orients the US device in relations of the subject's head. However, reliance on and reference to the Doppler images is operator dependent and, inevitably, different operators position the same US device on the same head differently, thereby causing quite significant discrepancies with reproducibility of the resulting transcranial insonation.

US patent application 2007/167765 A1 discloses an apparatus for transcranial sonothrombolysis with two opposed transducer arrays supported by a headset comprising positioning brackets.

In a case when the insonation of a specific clot of the cerebral vasculature is required, for example, not only the above-mentioned deviations and discrepancies with three-dimensional (3D) positioning of the US device at the head may result in imprecise insonation of the clot itself, but also the safety of the insonation procedure itself may be questioned. To counter such possibility, an operator is often required to conduct additional diagnostic study, which consumes even more time.

Furthermore, a sonothrombolysis procedure required that a target area be insonated for a prolonged period of time (in some cases, for a minimum of two hours), during all of which the conventional US device used for sonothrombolysis requires attention of the clinician / operator to be held in place on the subject's head.

Moreover, the insonation of the subject's head is traditionally carried out in a clinical setting and by the very nature of the used US devices does not allow or at least limits the transportation of the subject without interrupting the transcranial ultrasound procedure for sonothrombolysis, for example. There remains an unaddressed need, therefore, in a system and method for transcranial insonation that addresses these shortcomings and enables a repeatable positioning of the US on the head of a subject in orientation that is pre-fixed with respect to an identified portion of the cerebral vasculature, that is configured not to restrict the transporting or otherwise moving the subject during the insonation procedure, and that remains in place oriented for optimal insonation procedure without the input from the operator.

SUMMARY OF THE INVENTION

Embodiments of the present invention provide an apparatus, for transcranial sonothrombolysis of a subject's head, that includes a first temporal transducer array in cooperation with a first transducer-array registration member and a headset defining a headset plane, a headset surface, a front corresponding to a forehead of the subject's head, and a nasion registration member affixed to the headset at the front and protruding transversely to the headset plane. In the event when the subject suffers from a stroke, the apparatus of an embodiment is adapted for transcranial sonothrombolysis of a clot in subject's cerebral vasculature. Such headset is adapted to support the first transducer array on the headset surface (which, in one embodiment, is an internal surface of the headset facing the subject's head when the headset is positioned on the head for sonothrombolysis.) The headset includes anterior and posterior headframe members configured to mate at corresponding ends of these members such as to define said headset plane and a circumcranial loop of said headset. The mutual positioning of the anterior and posterior headframe members is adjustable (optionally, tensionably).

The nasion registration member is configured to rest on the nasion of the subject, when the headset is positioned on the subject's head for sonothrombolysis, such as to ensure that (i) the headset plane is inclined with respect to a reference plane defined by a left otobasion superior (OBS), a right OBS, and a nasion of the subject's head, and that (ii) the headset plane passes through said left and right OBS. The angle of inclination between the reference plane and the headset plane in such position is between about 10 degrees and about 15 degrees and, in a specific embodiment, about 12 degrees. The first transducer-array registration member is configured to align the first temporal transducer array with an otobasion superior and a sphenoid shelf of the subject's head, when the headset is positioned on the subject's head for sonothrombolysis, such as to optimize a degree of insonation of a target central cerebral vasculature with an ultrasound (US) beam emitted by said first transducer array substantially along the headset plane. The target central cerebral vasculature includes at least one of anterior cerebral artery (ACA), middle cerebral artery (MCA), posterior cerebral artery (PCA), internal carotid artery (ICA), basilar artery (BAS) and vertebral arteries (VER) and the Circle of Willis. An embodiment of the apparatus additionally includes an occipital transducer array affixed on the headset surface opposite to the nasion registration member and configured to emit an US beam substantially along the headset plane towards targeted cerebral vasculature. An apparatus optionally further contains a hand-held portable remote control unit configured to control an operation of at least one of the occipital and first temporal transducer arrays. Such remote control unit is operably connected to the headset either wirelessly or with an umbilicus. In one embodiment, the apparatus includes a second temporal transducer array cooperated with a second transducer-array registration member. In this case, the first and second transducer arrays are affixed on the headset opposite to one another such that, when the headset is positioned on the subject's head for transcranial sonothrombolysis, the second transducer-array registration member aligns the second temporal transducer array with an otobasion superior and a sphenoid shelf of the subject's head such as to optimize a degree of insonation of the target central cerebral vasculature with an US beam emitted by said second transducer array substantially along the headset plane.

Embodiments of the invention also provide an apparatus, for transcranial sonothrombolysis of targeted cerebral vasculature of a subject's head, that includes a set of transducer arrays (in a specific implementation - a temporal transducer array and an occipital transducer array) configured to be operated with a hand-held portable remote control unit; and a headset supporting such set of transducer arrays and mountable on the subject's head. The headset defines a headset plane and includes a set of mounting registration elements adapted to stereotactically cooperate with the subject's head such as to (i) align transducer arrays with corresponding transcranial acoustic windows of the subject's head when the headset is positioned on the subject's head for transcranial sonothrombolysis, and (ii) optimize an insonation of the targeted cerebral vasculature through said transcranial acoustic windows with US beams emitted by said transducer arrays in such stereotactically cooperated position. The mounting registration elements are adapted to orient the corresponding transducer arrays for emission of US beams substantially along the headset plane. The headset plane is inclined with respect to a reference plane (defined by chosen external craniological marks of the subject's head) when the headset is positioned on the subject's head for transcranial sonothrombolysis. Generally, the mounting registration elements includes a registration element configured to contact one of a nasion, left otobasion superior (OBS), right OBS, tragion, mandibular condyle, zygomatic arch, prosthion, and occipital prominence when the headset is positioned on the subject's head for transcranial sonothrombolysis. In a specific embodiment, the chosen external craniological marks include a left otobasion superior (OBS), a right OBS, and a nasion of the subject's head. In one embodiment, the mounting registration elements include a nasion registration bracket protruding transversely to the headset plane from a front of the headset and a temporal transducer array registration bracket slidably mounted on an internal surface of said headset. Such nasion registration bracket and the temporal transducer array registration bracket are configured, respectively, to be situated on the nasion of the subject and to engage an OBS when the headset is positioned on the subject's head for transcranial sonothrombolysis. In a specific implementation, the mounting registration elements are adapted to ensure that the headset plane passes through said left and right OBS when the headset is positioned on the subject's head for transcranial sonothrombolysis.

Optionally, the headset includes anterior and posterior headframe members configured to mate at corresponding ends of said members such as to define a circumcranial loop of said headset. The mutual positioning of the anterior and posterior headframe members is tensionably adjustable. An embodiment may additionally contain a hand-held portable remote control unit configured to power at least one of the transducer arrays, and, optionally, an umbilicus operably connecting the remote control unit to the headset.

Embodiments of the invention additionally provide an apparatus for transcranial sonothrombolysis of targeted vasculature of a subject's head, which apparatus includes one or more transducer arrays, a headset defining a headset plane and supporting said one or more transducer arrays, one or more brackets extending outwardly from the headset, wherein different brackets are used to position the headset with respect to different external craniological marks of a subject's head. In one case, the headset plane passes through the left and right OBS when the headset is positioned on the subject's head for transcranial sonothrombolysis.

One or more of these brackets of the apparatus are adapted to stereotactically cooperate with the craniological marks to position at least one of the transducer arrays to optimize an insonation of the targeted cerebral vasculature with ultrasound beams emitted by so positioned at least one of the transducer arrays. Moreover, at least one of the brackets is adapted to stereotactically align the at least one of the transducer arrays for transcranial sonothrombolysis, and to optimize an insonation of the targeted cerebral vasculature through one or more transcranial acoustic windows with the US beams. In a specific embodiment, the brackets include a nasion registration bracket protruding from a front of the headset and a transducer array registration bracket slidably mounted on an internal surface of the headset, and the external craniological marks include a left otobasion superior (OBS), a right OBS, and a nasion of the subject's head. The headset includes anterior and posterior headframe members configured to mate at corresponding ends of said members such as to define a circumcranial loop of said headset.

Embodiments of the invention also include a headset apparatus for providing transcranial sonothrombolysis of a target cerebral vasculature of a subject's head. The headset apparatus contains (i) a processor, (ii) a non-transitory tangible computer-readable medium with computer-readable program code encoded in this medium, and (iii) an ultrasound device that has a headset plane and that includes mounting registration brackets (defined with respect to corresponding external craniological marks of the subject's head) and transducer arrays. The headset apparatus is positionable onto the subject's head such that the mounting registration brackets are brought into contact with respectively corresponding external craniological marks and the headset plane is placed at an angle with respect to a reference plane defined by the external craniological marks, The computer-readable program code includes a series of computer-readable program steps to effect (a) insonating, in the headset plane, the target cerebral vasculature; and (b) defining a regime of operation of the at least one of the transducer arrays of the headset apparatus. In one embodiment, the step of insonating includes insonating with at least one of a temporal transducer array and an occipital transducer array and, in addition or alternatively, the step of defining includes defining at least one of temporal and amplitude sequence of pulses of US beams.

Described herein is also a method for transcranial sonothrombolysis of a subject's head with a ultrasound US device having a headset that defines a headset plane and that contains a transducer array and mounting registration elements including a nasion registration member and at least one temporal registration member. (In one embodiment the headset contains a temporal transducer array, an occipital transducer array, and a hand-held portable remote control unit with a processor pre-programmed to define a regime of operation of at least one of the temporal and occipital transducer arrays.) In the event when the subject suffers from a stroke, the method of an embodiment is adapted for transcranial sonothrombolysis of a clot in subject's cerebral vasculature. The method for sonothrombolysis includes positioning the headset onto the subject's head such that (i) the nasion registration member rests on the nasion of the subject and (ii) the headset plane is inclined with respect to a reference plane defined by a left otobasion superior (OBS), a right OBS, and the nasion and (iii) the headset plane intersects the reference plane substantially at left and right OBS. The method further includes insonating targeted cerebral vasculature that is defined by so inclined headset plane. The positioning of the headset may include establishing contact between the at least one temporal registration member and an OBS and, alternatively or in addition, positioning the headset such as to incline the headset plane with respect to said reference plane by an angle within a range from about 10 degrees to about 15 degrees. The insonation includes a process of insonating at least one of anterior cerebral artery (ACA), middle cerebral artery (MCA), posterior cerebral artery (PCA), internal carotid artery (ICA), basilar artery (BAS) and vertebral arteries (VER) and the Circle of Willis with an US beam emitted by the transducer array.

Described herein is also a method for transcranial sonothrombolysis of target cerebral vasculature of a subject's head with an ultrasound (US) device having a headset, which defines a headset plane and includes a set of mounting registration elements, and transducer arrays including a temporal transducer array and an occipital transducer array. The method includes choosing such external craniological marks associated with the subject's head that define the location of said target cerebral vasculature; and defining mounting registration elements on the headset that respectively correspond to the chosen external craniological marks. (The definition of mounting registration elements includes defining a nasion registration bracket protruding transversely to the headset plane and configured to be situated at the nasion of the subject when the head is positioned on the subject's head for transcranial sonothrombolysis, and defining a registration member associated with the temporal transducer array and configured to be in contact with an otobasion superior when the head is positioned on the subject's head for transcranial sonothrombolysis).

The method additionally includes positioning the headset on the subject's head such as to establish contact between the defined mounting registration elements and the respectively corresponding external craniological marks. The method further includes activating a transducer array to insonate the target cerebral vasculature substantially along the headset plane, which activation is optionally enabled with the use of a hand-held portable remote control unit (either wirelessly or through a connecting umbilical cable or umbilicus). The process of choosing external craniological marks includes choosing the craniological marks that define a reference plane across the subject's head and, in a specific implementation, choosing at least three of a nasion, left otobasion superior (OBS), right OBS, tragion, mandibular condyle, zygomatic arch, prosthion, and occipital prominence.

Described herein is also a computer program product for providing transcranial sonothrombolysis of a target cerebral vasculature of a subject's head with an ultrasound device having a headset (which identifies a headset plane and includes a set of mounting registration elements defined with respect to corresponding external craniological marks of the subject's head) and transducer arrays, wherein the headset is configured to be positionable onto the subject's head such that mounting registration elements are brought into contact with respectively corresponding external craniological marks. Such computer program product includes a tangible computer-usable medium having computer-readable program code thereon, the computer-readable program having (i) program code for activation at least one of the transducer arrays such as to optimize an insonation of the target cerebral vasculature substantially in the headset plane; and (ii) program code for defining a regime of operation of the at least one of the transducer arrays. Furthermore, the program code for defining a regime of operation optionally includes program code for such insonation of the target cerebral vasculature that a peak rarefaction pressure does not exceed 300 kPa and a thermal index does not exceed a physiologically compatible thermal index at a predetermined depth within the subject's head. Alternatively or in addition, program code for defining a regime of operation includes program code adapted to cause emission of US pulses by the at least one of said transducer arrays at a pulse interval that is determined to prevent echoes from constructive interference between said US pulses and outgoing pulses. In a specific embodiment, such pulse interval is between 150 milliseconds and 300 milliseconds.

BRIEF DESCRIPTION OF THE DRAWINGS

The invention will be more fully understood by referring to the following Detailed Description of Specific Embodiments in conjunction with the Drawings, of which:

Fig. 1 is a perspective view of an US device according to an embodiment of the invention.

Figs. 2A and 2B are side and plan views of a headset portion of an embodiment of the invention.

Fig. 3 is a rendering of a head with selected craniological landmarks of the skull that have a spatial relationship with the sphenoid shelf (and anterior clinoid processes) on which sits the Circle of Willis. Three such landmarks are used to stereotactically align the arrays with acoustic windows and cerebral vasculature, according to an embodiment of the invention.

Fig. 4A is an exposed view of the cranial bones forming the sphenoid shelf and of the associated cerebral arteries.

Figs. 4B and 4C are, respectively, superior and lateral views of the major cerebral arteries and the Circle of Willis, which can be seen resting tangential to the anterior and posterior clinoid processes and projecting in plane from the shelf or ledge formed by the greater and lesser wings of the sphenoid bone (or "sphenoid shelf") that forms the base of the anterior fossae and overlies and is generally co-planar with the orbital tracts.

Fig. 5A is a diagram illustrating the triangular foundational (or reference) plane defined by mounting registration marks and used, according to an embodiment of the invention, to orient the headset so that the transducer arrays are positioned and aligned in optimal registration with the cerebral vasculature.

Fig. 5B illustrates a fixed inclination of the headset of an embodiment relative to the foundational registration plane of Fig. 5A.

Fig. 6 is a side view of the headset on a user showing an embodiment of the invention mounted thereon.

Fig. 7A is an internal view of the headset with a nasion registration bracket and a pad configured for aligning the transducer arrays with the nasion and the sphenoid shelf.

Fig. 7B and C are perspective views of the temporal transducer array subassemblies.

Fig. 7D is a cutaway view showing detailed structure of the temporal transducer array and a transducer-array housing.

Fig. 8A depicts an exposed view of the temporal transducer array in cooperation with a temporal acoustic window on the ipsilateral side of the skull. The view of the targeted central cerebral vasculature is overlapped with the view of the transducer array.

Fig. 8B is a cross-sectional exposed view showing the ultrasound beams directed from the temporal transducer array towards the targeted cerebral vasculature.

Fig. 9A is a top view of the headset assembly, showing temporal and occipital ultrasound beams directed from corresponding transducers towards the targeted cerebral vasculature.

Fig. 9B is a cutaway side view of an embodiment of the device depicting ultrasound beams of the occipital transducer array directed at the targeted cerebral vasculature.

Fig. 9C is a perspective view illustrating a hand-held portable remote control unit for use with an embodiment of the invention.

Figs. 11A and 11B are plots illustrating regimes of pulsed operation of a transducer of an embodiment of the invention.

Fig. 12A is an example of a metapulse cycle of operation of an embodiment of the invention that includes sixteen US transducers. Combining the traces, the figure represents a pattern of asynchronous ultrasonic pulse train emissions (i.e., a "metapulse cycle"), where each single crystal is directed at a distinct anatomical target and is fired once per cycle.

Fig. 12B is another example of a metapulse cycle, here having duplex, paired transducer firings.

Fig. 12C is yet another example of a metapulse insonation cycle, here having triplex simultaneous transducer firings.

Fig. 13 quantifies the audible sensation of ultrasound exposure as a function of modulated pulse train frequency.

Fig. 14A is a plot illustrating peak rarefaction pressure ($P_r$) as a function of depth for selected increments in transducer voltage ($V_{p-p}$).

Fig. 14B is a plot illustrating the relationship between peak rarefaction pressure and ICH conversion (%) empirically determined based on analysis of data from clinical trials.

Fig. 15A is a plot of peak rarefaction pressure illustrating the effect of thick and thin skull phenotype on peak rarefaction pressure as a function of depth at 1 MHz.

Fig. 15B is a curve fit for attenuation coefficient ($A_{TempBONE}$) versus frequency for temporal bone.

Figs. 16A and 16B are renderings of physical models for analysis of attenuation profile as a function of depth for a trans-temporal transducer (Fig. 16A) and for a transducer apposing a non-boney, sub-occipital acoustic window (Fig. 16B).

Fig. 17 is a flow-chart (390) depicting automated operation of an apparatus of the invention where emission voltage is adjusted to detect acoustic coupling of each of the used transducers.

Figs. 18A is a diagram describing the use of phase angle to verify acoustic coupling.

Fig. 18B is a plot of voltage output versus phase angle corresponding to an embodiment of the coupling verification circuit of Fig. 19.

Fig. 19 illustrates schematically a coupling verification circuit (400) which relies on a voltage comparator having the output of Fig. 18B.

Figs. 20A and 20B are diagrams depicting a model for vascular vasodilation with release of endogenous nitric oxide, where blood shear (Fig. 20A) is replaced by noninvasive ultrasound from an apparatus of the invention (Fig. 20B).

## DETAILED DESCRIPTION

In accordance with preferred embodiments of the present invention, apparatuses are disclosed configured for a non-invasive transcranial ultrasound procedure that is devoid of imaging-guided placement of the US device, in cooperation with the subject's head, that is required by currently employed methods and apparatus of related art. An embodiment of the system of the invention includes stereotactic positioning structure(s) and is adapted for positioning onto the subject's head in a pre-determined reproducibly achievable orientation, which positioning ensures that a specified element of the subject's cerebral vasculature is reliably insonated according to a method of the invention.

References throughout this specification to "one embodiment," "an embodiment," "a related embodiment," or similar language mean that a particular feature, structure, or characteristic described in connection with the referred to "embodiment" is included in at least one embodiment of the present invention. Thus, appearances of the phrases "in one embodiment," "in an embodiment," and similar language throughout this specification may, but do not necessarily, all refer to the same embodiment. It is to be understood that no portion of disclosure, taken on its own and in possible connection with a figure, is intended to provide a complete description of all features of the invention.

In addition, the following disclosure may describe features of the invention with reference to corresponding drawings, in which like numbers represent the same or similar elements wherever possible. In the drawings, the depicted structural elements are generally not to scale, and certain components are enlarged relative to the other components for purposes of emphasis and understanding. It is to be understood that no single drawing is intended to support a complete description of all features of the invention. In other words, a given drawing is generally descriptive of only some, and generally not all, features of the invention. A given drawing and an associated portion of the disclosure containing a description referencing such drawing do not, generally, contain all elements of a particular view or all features that can be presented is this view, for purposes of simplifying the given drawing and discussion, and to direct the discussion to particular elements that are featured in this drawing. A skilled artisan will recognize that the invention may possibly be practiced without one or more of the specific features, elements, components, structures, details, or characteristics, or with the use of other methods, components, materials, and so forth. Therefore, although a particular detail of an embodiment of the invention may not be necessarily shown in each and every drawing describing such embodiment, the presence of this detail in the drawing may be implied unless the context of the description requires otherwise. In other instances, well known structures, details, materials, or operations may be not shown in a given drawing or described in detail to avoid obscuring aspects of an embodiment of the invention that are being discussed. Furthermore, the described single features, structures, or characteristics of the invention may be combined in any suitable manner in one or more further embodiments.

Moreover, the schematic flow chart diagram included is generally set forth as a logical flow-chart diagram. As such, the depicted order and labeled steps of the logical flow are indicative of one embodiment of the presented method. Other steps and methods may be conceived that are equivalent in function, logic, or effect to one or more steps, or portions thereof, of the illustrated method. Additionally, the format and symbols employed are provided to explain the logical steps of the method and are understood not to limit the scope of the method. Although various arrow types and line types may be employed in the flow-chart diagrams, they are understood not to limit the scope of the corresponding method. Indeed, some arrows or other connectors may be used to indicate only the logical flow of the method. For instance, an arrow may indicate a waiting or monitoring period of unspecified duration between enumerated steps of the depicted method. Without loss of generality, the order in which processing steps or particular methods occur may or may not strictly adhere to the order of the corresponding steps shown.

The invention as recited in claims appended to this disclosure is intended to be assessed in light of the disclosure as a whole.

Each year in the United States about 700,000 strokes occur, and in more than 150,000 of such cases strokes happen to be lethal, while in most of the remaining cases strokes are debilitating for those who survive. Importantly, centralized stroke clinical care centers are currently attending only about 3% of stroke cases, and mortality and morbidity following advanced diagnosis and treatrnent-absent sonothrombolysis procedure -have improved by only 20% overall even with the most advanced care.

Tools for non-invasive sonothrombolysis, as known in the art, remain experimental, and have not yet resulted in changes to the basic standard of care for stroke or dramatically improved the prognosis. The use of existing sonothrombolysis devices suffers from significant imprecision of insonation of a target area of the head of a subject (due to non-repeatable and operator-dependent positioning of such devices in cooperation with the subject's head) and non-portability (due to requirement for a stationary power supply). Accordingly, a long-felt need for an apparatus for stroke care that has improved efficacy, is non-invasive, non-surgical, and safe has not been met.

Generally, embodiments of the present invention include an US sonothrombolysis device for transcranial insonation that is formatted as a headset defining a headset plane (and configured to be mounted circumcranially) and that includes a set of registration structural features (also referred to as a registration system) facilitating positioning of transducer arrays of such US device stereotactically or, in other words, in relation to an external frame of reference.

*Registration System in relations to External Craniological Marks.*

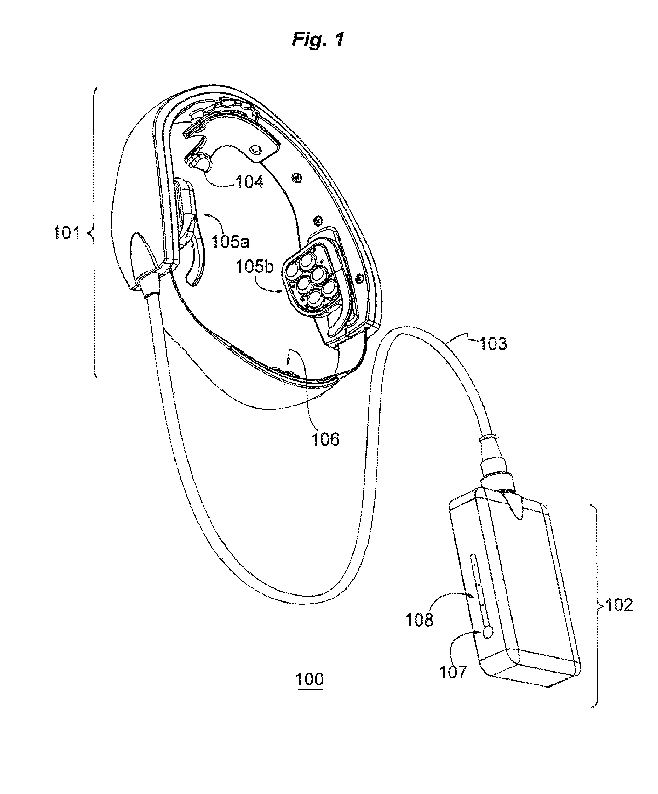

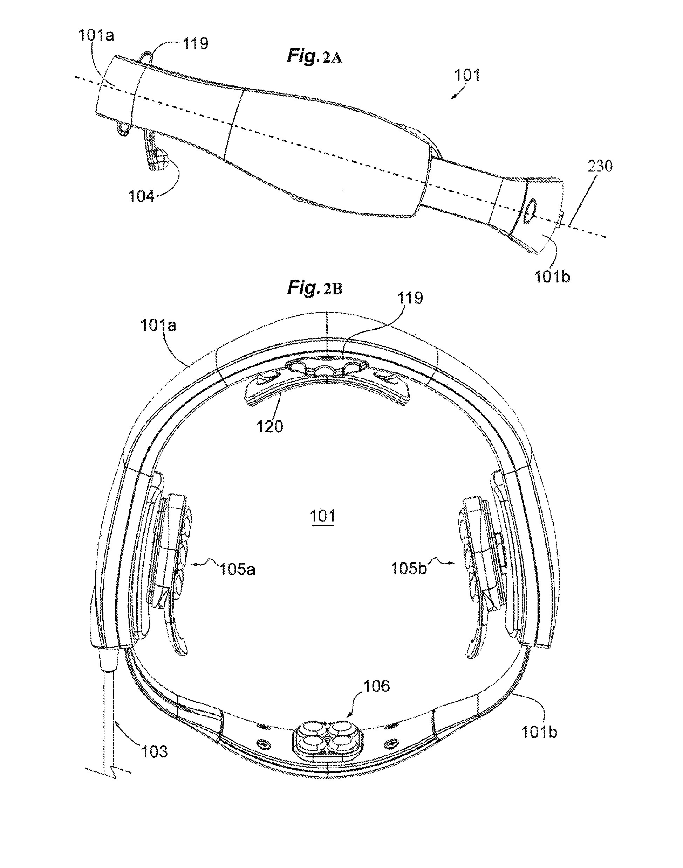

Figs. 1, 2A, and 2B provide a general description of an embodiment of the US sonothrombolysis device of the invention. Fig. 1, for example, is a perspective view 100 of such sonothrombolysis device. A shown, the embodiment 100 includes a headset or headset assembly 101 defining a headset plane and configured for circumcranial positioning in cooperation with a subject's head, a remote control unit 102 with power supply unit in a pocket-sized housing, and a cable or umbilicus 103 that operably and, optionally, detachably connects the headset 101 and the remote control 102. (In a related embodiment, not shown, the remote control unit 102 is operably connected to the headset 101 wirelessly. In another related embodiment, elements of the remote control unit that are described below are cooperated with or integrated into an element of the headset assembly 101.) The remote control unit 102 is supplied with appropriate operational elements such as, for example, an on/off or pause actuator or button 107 and a status indicator bar 108. The headset 101 is designed for extended periods of wear and weighs less than 500 grams, with the overall embodiment 100 weighing less than 1 kg. The embodiment 100 is shown to include three transducer arrays repositionably affixed to an internal side of the headset 101 (two temporal transducer arrays 105a, 105b and an occipital transducer array 106), but more generally includes a different number of multiple transducer arrays. In one implementation, for example, the embodiment 100 contains 6 transducers in each of the temporal arrays 105a, 105b and 4 transducers in the occipital array 106. Each of the transducer arrays 105a, 105b, 106 contains a plurality of piezoelectric crystals, each about 1 cm in diameter, that are optionally individually powered and operate, generally, within a range from about 500 kHz to about 3500 kHz and, in a specific embodiment, between about 1 MHz and about 2 MHz. In one implementation, the structural shell and/or supporting elements of the headset assembly 101 are constructed of a radiolucent plastic material having low Hounsfield density. Examples of such plastic materials include polycarbonate, acrylonitrile butadiene styrene (ABS), styrene-acrylonitrile, polystyrene, nylons, polyethylenes, acrylates, and so forth. For example, Bayblend (Bayer MaterialScience, Dusseldorf DE) may be used in construction of headsets of the invention, although not limited thereto. The CT translucency of various plastics has been described, for example, by Henrikson (in CT evaluation of plastic intraocular foreign bodies, Am. J. Neuroradiology, 8:378-79, 1987).

Figs. 2A and 2B provide side and top views of the headset assembly 101 of Fig. 1 showing elements of the stereotactic registration or positioning system (such as nose and ear registration brackets) configured to align the transducers of the embodiment with the targeted acoustic windows in the skull and the targeted cerebral vasculature, as discussed below, when the headset 101 is cooperated with the subject's head. The above-mentioned headset plane defined by the headset 101 is a plane 230 extending along the headset 101 and intersecting the plane of Fig. 2A along a dot-dashed line as shown. Specifically, the headset assembly 101 includes an arcuate anterior headframe member 101a adapted to fit to the front of the subject's head and having two open ends configured to receive the corresponding ends of an arcuate, pliant, posterior headframe member 101b. As shown, the member 101a and 101b are mated in that the ends of the member 101b are inserted into the corresponding ends of the member 101a to define, in the mated position, a plane of the headset 101 and a circumcranial loop of the headset 101 to be fitted around the subject's head. Mutual positioning of the members 101a and 101b and the size of the loop of the headset 101 are adjustable by means of a tightening mechanism or knob 119 within the body of the anterior headframe member 101a. As shown, the knob 119 is disposed at a front of the headset 101 (which corresponds to a forehead of the subject's head) but can generally be disposed at a different location. Tightening is achieved by tensioning the posterior headframe member 101b and does not alter or otherwise affect circumcranial placement of the anterior headframe member 101a with registration brackets, thus ensuring that the stereotactic alignment with respect to the cerebral vasculature is not disturbed. The headset 101 further includes a set of registration system elements (discussed in detail below) one of which is a nasion registration bracket 120. The bracket 120 is mounted anteriorly inside the headframe 101 and supporting a nosepad 104 that is adapted to be cooperated with and be fitted against the nasion on the bridge of the subject's nose.

As mentioned, according to the idea of the invention, the registration system of the US device of the invention is structured to ensure that, once the embodiment of the device is cooperated with the subject's head, the transducers of the embodiment are reliably, in a single move, positioned and oriented in a particular, pre-determined spatial relationship with the features of skull thereby enabling the transducers to predictably emit ultrasound towards the targeted cerebral arteries most commonly associated with stroke. This is in stark contradistinction with prior art solutions that require re-iterative operator-involved reorientation of the arrays to achieve the optimal positioning of the arrays on the subject's head and, therefore, delay the initiation of an insonation procedure. One of the arterial targets of insonation includes, for example, the brain's arteries of the so-called Circle of Willis, the cerebrovascular tree of which creates redundancies or collaterals in the cerebral circulation. (The Circle of Willis is interchangeably referred to herein as a cerebral arterial nexus.) In particular, if one part of the Circle becomes blocked or narrowed (stenosed) or one of the arteries supplying the circle is blocked or narrowed, blood flow from the other blood vessels can often preserve the cerebral perfusion well enough to avoid the symptoms of ischemia. At the same time, statistically, many strokes are found to be strokes of the cerebral arteries associated with the Circle of Willis. Therefore, (i) defining the orientation of the Circle of Willis in relation to external craniological marks, and (ii) configuring the registration system of the US device to set the spatial positioning of the US device, with respect to the same external craniological marks, that relies on such defined orientation ensures that the transducers of the US device are positioned unambiguously, passively (i.e., without the need or use of any feedback characterizing the precision of such positioning, be it the feedback from a clinician or a machine), and repeatedly (i.e., any time when the US device is cooperated with a subject's head) for insonation of the of the Circle of Willis and related cerebral arteries.

The registration system of an embodiment of the invention is defined by external craniological marks associated with a human head. The repeated use of such conserved external craniological marks eliminates a need, for a trained clinician and/or sonographer, to adjust and re-adjust the headset of the device on the subject's head. According to embodiments of the invention, an external frame of reference for sonothrombolysis includes external osteology, where the cranial frame of reference is based on one or more craniological marks of the head such as, for example, nasion, left otobasion superior, right otobasion superior, tragion, mandibular condyle, zygomatic arch, prosthion, and occipital protuberance. In one embodiment, for example, a triangular frame of reference is used for defining a registration system of the US device. Such triangular frame of reference includes at least three marks such as the nasion and the right (Rt) and left (Lt) otobasion superiora marks. The choice of such triangular frame of reference unambiguously establishes the relative positions of (i) the temporal and sub-occipital acoustic windows in the subject's skull, (ii) the sphenoid "shelf" formed by the greater and lesser wings of the sphenoid bone, (iii) the anterior and posterior clinoid processes, (iv) dorsum sellae, and (v) the Circle of Willis with cerebral arterial circle, bifurcations of the internal carotid artery conjoining the anterior, middle and posterior cerebral arteries, and junctures of the basilar artery with the communicating cerebral arteries and the vertebral arteries. Conventional frames of reference to a subject's head (such as reference planes) chosen by the related art are defined to orient the US device with respect to either a vertical plane or a horizontal plane. The elements of cerebral vasculature that are targeted by the system and method of the present invention - such as the Circle of Willis, for example - define a plane that is neither vertical nor horizontal but rather is inclined with respect to a horizontal plane. Therefore, a conventionally-defined insonation procedure or a US device, the operation of which is defined with respect to a conventional frame of reference, are not configured to reproducibly target and insonate the Circle of Willis, for example. Accordingly, the frame of reference defined by embodiments of the present invention was empirically proven to be more reliable than the conventional Broca's reference plane (also known as the "neuro-ocular plane" or NOP), and is much preferred over the Frankfurt-Virchow plane, which lies oblique to and below the target anatomy. While the NOP, which follows the orbital tracts, is slightly below and parallel to the Circle of Willis, its use requires a measurement of 3.3 cm above the tragion and is not easily available to an unskilled person. In contradistinction, the use of the frame of reference defined by embodiments of the present invention does not require any instructions and/or preparation of the clinical personnel. As will be discussed further, the required spatial orientation of the device with respect to the pre-defined reference plane is preset by design of the headset geometry.

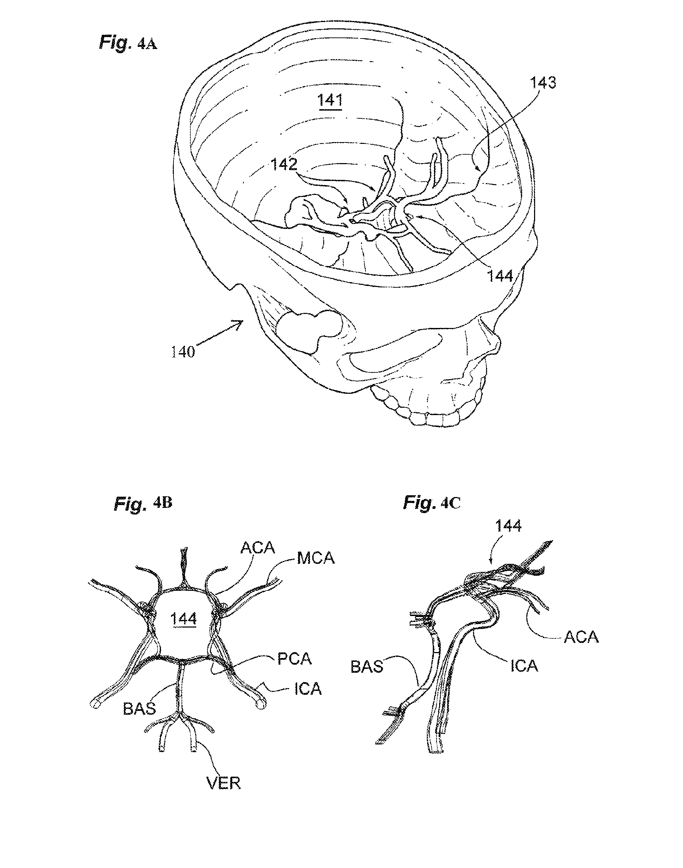

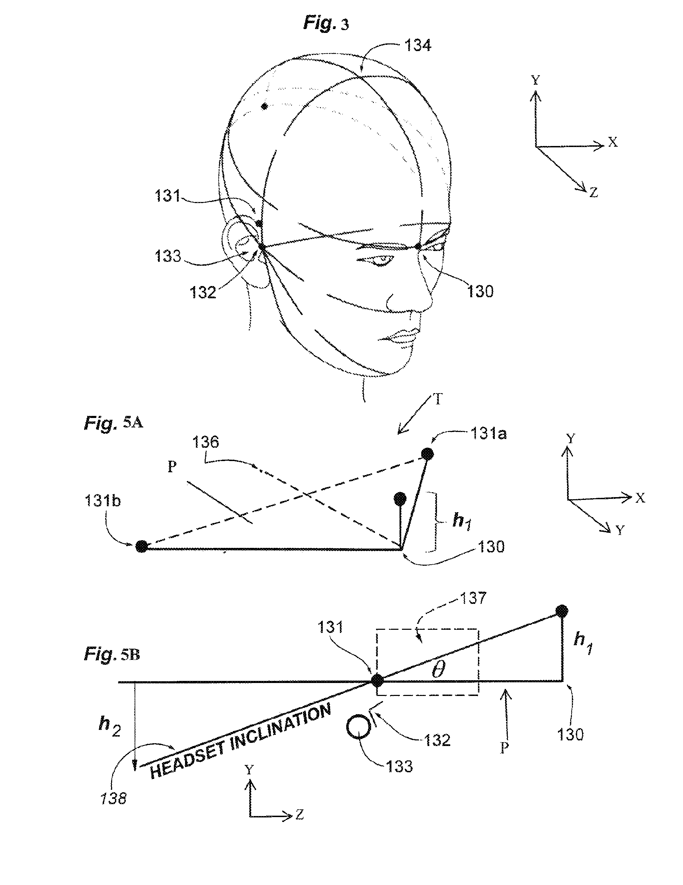

To facilitate the understanding of the spatial cooperation of an embodiment of the invention with a subject's head, Figs. 3, 4A, 4B, and 4C provide illustrations of the mutual orientation of the targeted portion(s) of the cerebrovascular system and the external system of craniological marks that define a registration system of an embodiment of the invention. In particular, Fig. 3 is a diagram providing a rendering of a head with characteristic external craniological marks, of the head, having spatial relationships with the sphenoid shelf (marked by the anterior clinoid processes, see Fig. 4A) on which a major portion of the Circle of Willis is located. Among the external craniological marks, chosen in accord with an embodiment of the invention to define a registration system of a device of the invention, there are nasion 130, otobasion superior or OBS (a shown, right OBS 131), tragion 132, auditory meatus (with the center auricular point, 133), brema 134, as well as mandibular condyle, zygomatic arch, prosthion, bregma, or occipital prominence (OCP) (not indicated). Fig. 4A is a perspective exposed view of the cranium 140, showing the cranial vault 141, bones (such as greater and lesser wings of the sphenoid bone) forming an anatomical feature termed herein "the sphenoid shelf' 143, and the general position of the major cerebral arteries 142, 144. Figs. 4B and 4C are superior and lateral views of the major cerebral arteries and the Circle of Willis 144. Shown are the anterior cerebral artery (ACA), middle cerebral artery (MCA), posterior cerebral artery (PCA), internal carotid artery (ICA), basilar artery (BAS) and vertebral arteries (VER) and their connections to the Circle of Willis (144). Referring again to Fig.4A, the Circle of Willis 144 is shown to be resting partially on the sphenoid shelf 143 that forms the base of the anterior fossae overlying the orbital tracks and is generally co-localized with the Alleman plane.

According to the idea of the invention, at least three of external registration marks are used to stereotactically align the headset of an embodiment of the US device (such as, for example, the headset 101 of Fig. 1) and the corresponding arrays of transducers (such as the arrays, 105, 105b, 106 of Fig. 1) with acoustic windows in he skull and the targeted cerebral vasculature. In one implementation, for example, the chosen marks include the nasion 130, and the left and right OBS. To this end, Fig. 5A shows a diagram illustrating a reference plane P (the *xz*-plane in Fig. 5A, also referred to herein as Alleman Plane) containing the chosen registration marks (the nasion 130, the left OBS 131a, and the right OBS 131b). The Isosceles triangle T, defined by the marks 130, 131a, 131b, has a base line connecting the left and right OBS 131a, 131b and is bisected by a straight midline136. The midline 136 extends anteriorposteriorly through the cranial vault generally tangentially to the sphenoid shelf 143 of Fig. 4A and parallel to and slightly above the optical tract of the subject (not shown), and defined the cerebral hemispheres. According to an embodiment of the invention, the reference Alleman plane defined by the triangle T is an external guide to the location of the cerebral vasculature that is most susceptible to stroke.

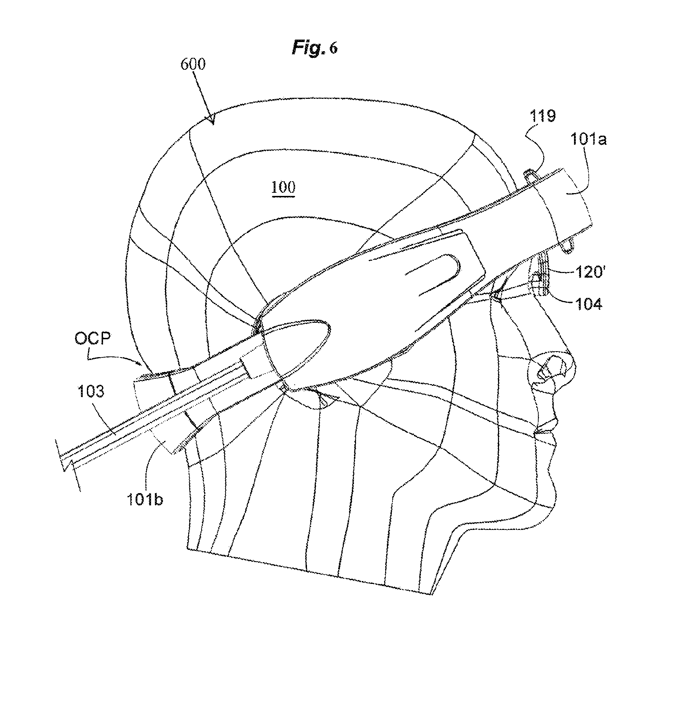

As shown in a diagram Fig. 5B, for example, the headset of the US device is configured to be inclined, in one implementation, with respect to the Alleman plane P once the headset is placed onto the subject's head (as shown, a line "headset inclination" 138, which, in further reference to Fig. 2A, substantially coincides with the headset plane 230 in this position of the headset 101.). Such inclination is achieved, for example, by structurally offsetting the anterior member of the headframe 101 a of the headset above the nasion 130, for example by a distance $h_1$. It is recognized that the height of the glabella in the adult is about 0.7 to about 1.6 centimeters. Therefore, if the structural offset distance (height) $h_1$ is chosen to be about 2 centimeters, the anterior member of the headset 101a clears the brow of the wearer. The posterior headband member 101b is structurally adapted to be seated below the occipital protuberance by a distance $h_2$. When so inclined, the headset continues to rest on the external registration marks. The inclination angle $\theta$ is chosen to be between about 10 to about 15 degrees, and preferably about 12 degrees. Fig. 6 is a side view of an embodiment 100 of Fig. 1 that is disposed, according to the spatial relationship described in reference to Figs. 3, 4A, and 4B, on the subject's head 600 and cooperated with the chosen external registration marks. The temporal transducer arrays 105a, 105b of the embodiment 100 may then be slideably positioned on the headframe over the temporal acoustic window 137 of the skull shown in Fig. 5B.

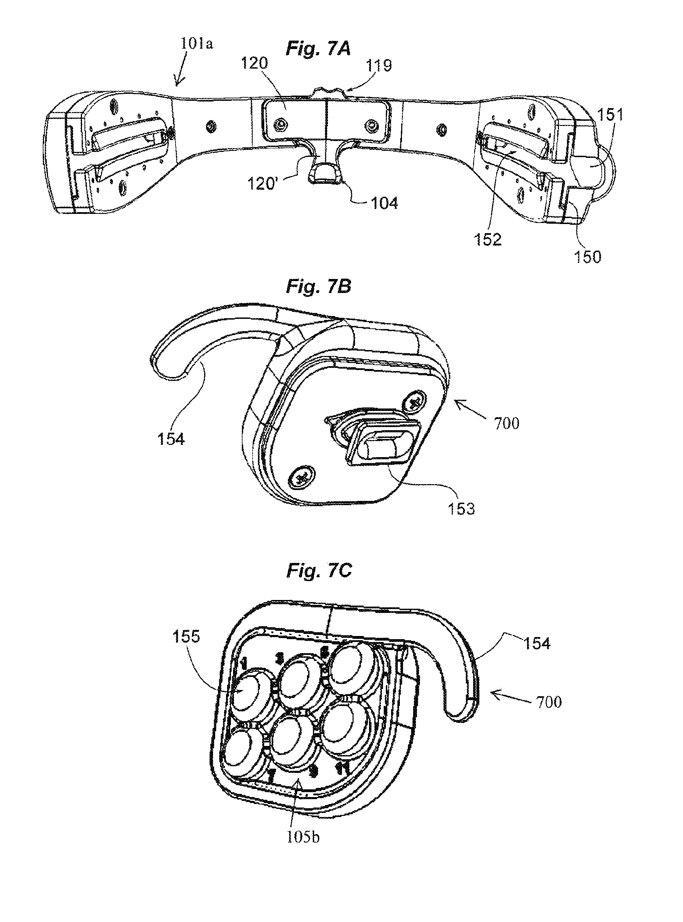

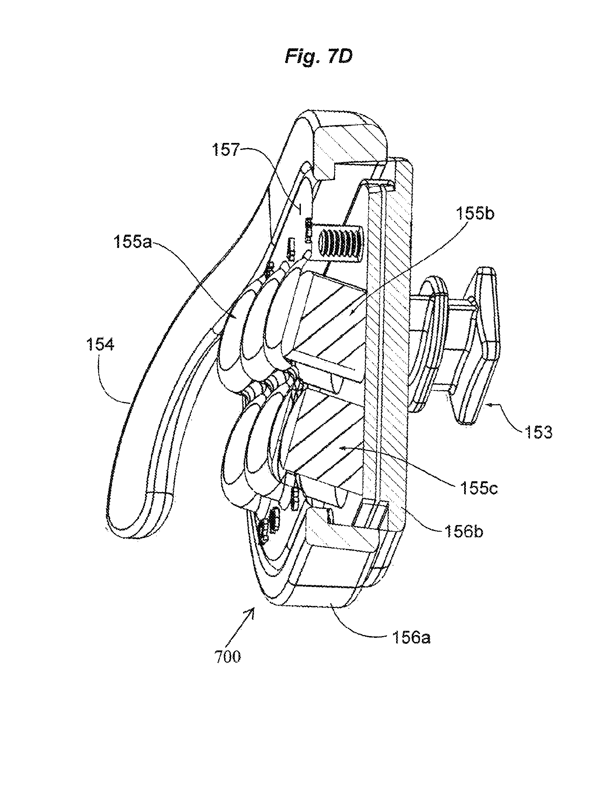

To enable and effectuate such required inclination and offset with respect to the external craniological marks, an embodiment of the invention includes mechanical brackets having appropriate surfaces configured to engage the chosen external marks when the headset of the embodiment is placed on the subject's head. Figs. 7A, 7B, 7C, and 7D provide illustrations of portions of the headset 101 of Fig. 1 with elements of the registration system configured to enable the predetermined positioning of the US device of the invention with respect to the registration marks (that were discussed in reference to Figs. 3, 4A, 4B, and 6).

One of the elements of the registration system of the invention, the nasion registration bracket 120, is shown in the back elevational view of the anterior headframe member 101a in Fig. 7A and the views of Figs. 7B through 7D. Two additional elements of the registration system, specifically, lateral registration OBS brackets, are represented by a lateral registration OBS bracket 154 of a right subassembly 700 including the temporal transducer array 105b. The lateral registration OBS bracket 154 (formatted as an earpiece) includes a surface 153 that is used to align the temporal transducer array with the otobasion superior and the sphenoid shelf 143 shown Fig. 4A. A slider foot 153 of the subassembly 600 is appropriately dimensioned to be inserted and slidably and securely fit into the right slider track 152 of the headframe member 101a, thereby permitting fitted positioning of the transducer array 105b anterior to the ear of the subject so that the transducer array engages the temporal acoustic window 137 of Fig. 5B when the fully assembled headset 101 is positioned on the subject's head. Referring again to Fig. 5A, such positioning defines an anterioposterior adjustment of the subassembly 700 in relation to the midline and the base of the triangle T. (A counterpart of the subassembly 700 is configured analogously to the subassembly 600, contains the second transducer array 155a, and is cooperated with the headframe 101a of Fig. 7A via the second slider track of the left side of the headframe 101a to be anterioposteriorly adjusted in relation to the midline and base of the triangle Ton the other side of the subject's head. This counterpart subassembly is not shown for simplicity of illustration).

Fig. 7D is a diagram showing, in a perspective view, the embodiment 700 with a partial cross-section through piezoelectric crystals of the transducers 155b, 155c of the temporal array 105b. Transducers 155a, 155b, 155c (shown arranged in a six unit array) are optionally contained in a housing 156a and covered by a baseplate 156b that is cooperated with the slider foot 153. The housing includes a thin overlayer 157 adapted as a coupling layer for more efficiently coupling acoustic output of the transducers 155a, 155b, 155c to the skull. The overlayer 157 may include polyurethane, polyethylene, silicon, rubber, and similar materials that are rather soft and compliant and the value of hardness of which lies between the hardness of the transducer and the hardness of the skull. Alternatively or in addition, the overlayer 157 may include a couplant gel known in the art. Referring again to Fig. 6, when the headset 101 with the anterior headframe member 101a carrying, on its internal surface, a subassembly 700 and its counterpart is placed on the subject's head, the subassembly 700 and its counterpart are generally aligned with and above the upper border of the zygomatic arch so that the corresponding transducers 155a, 155b, 155c are in contact with the head and acoustically coupled, through the thin overlayer 157, to the temporal acoustic window 137 of Fig. 5B. The temporal acoustic window 137 is defined by a location of a portion of the skull having a bone that is thinner in comparison to a bone in another portion of the skull.

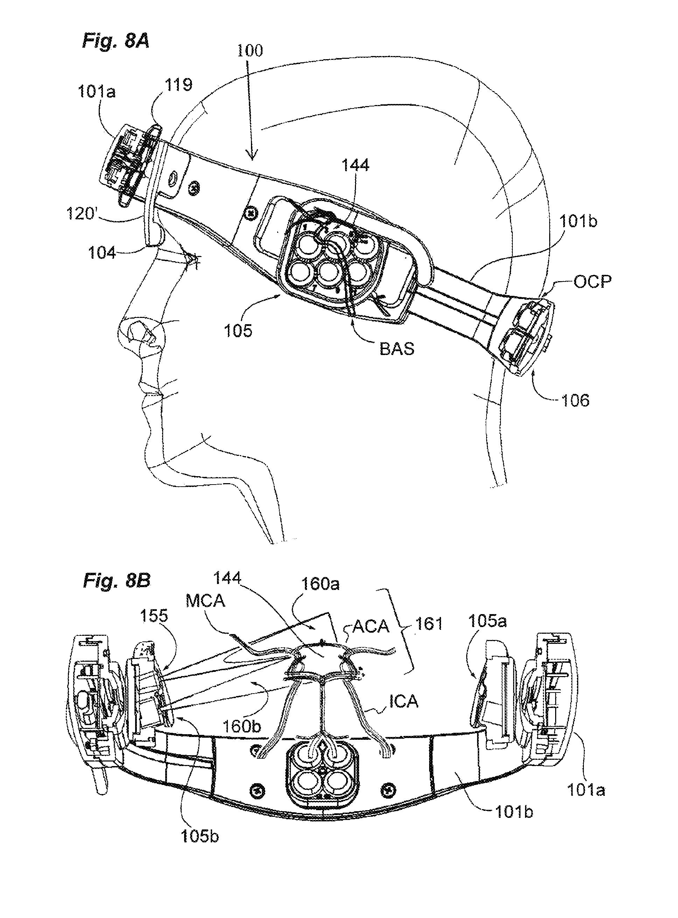

Fig. 8A depicts a temporal transducer array 105 (of the temporal transducer arrays 105a, 105b) as it is adapted to interface with the temporal acoustic window on the ispilateral side of the skull, as seen from a superimposed view of the transducer arrays and central cerebral vasculature (visible are BAS and the Circle of Willis 144). Also shown is the occipital transducer array 106 anchored under the OCP. The nasion registration riser or bracket 120' with the beveled pad 104, a posterior headframe member 101b secured under the OCP, and the operation of the tensioning knob 119 facilitate the stabilization of the unique and unambiguously defined anatomical position of the headset 101 with respect to the chosen craniological marks. With the headset 101 oriented this way, the head may be bent forward to open the occipital acoustic window without loss of stereotactic positioning of the tight (Rt) and left (Lt) temporal transducer arrays 105a, 105b. Fig. 8B is a perspective illustration depicting the orientation of the non-focused ultrasound beams (shown schematically as 160a, 160b) emitted by the Rt temporal transducer array 105b with respect to the internal anatomy of the targeted cerebral vasculature. The acoustic beams propagate through the temporal acoustic window 137 (shown in Fig. 5B), and on, 161, through the major nexii of the cerebral vasculature including the MCA, ACA, ICA and the Circle of Willis 144. In one implementation, the Rt and Lt temporal transducer arrays 105b, 105a are configured to emit ultrasound in alternation to irradiate/insonate the hemispheres of the brain substantially equally.

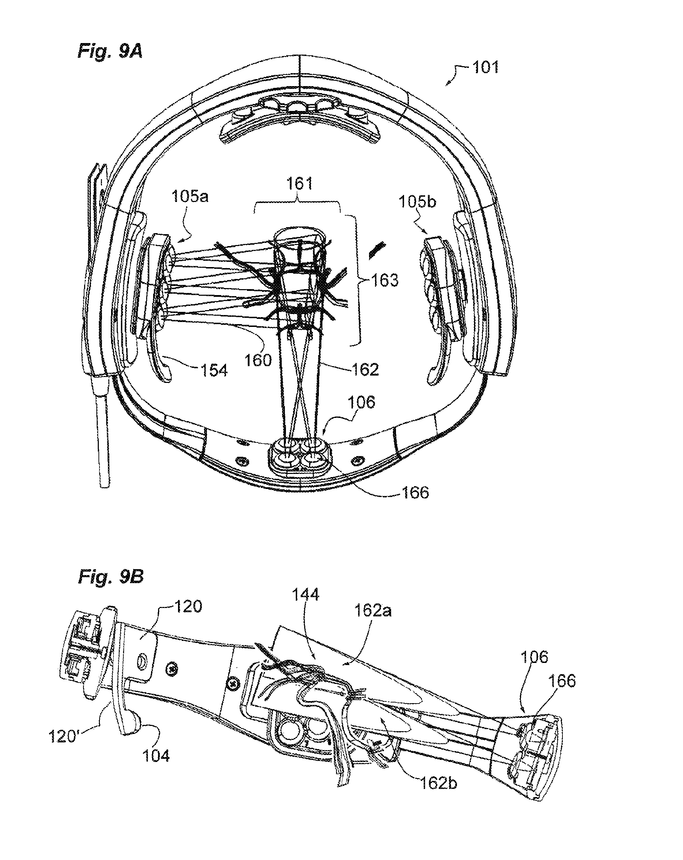

Figs. 9A, 9B provide additional illustrations of an implementation of mechanical registration between the embodiment of invention and the targeted cerebral vasculature with the use of chosen external craniological marks. Fig. 9A, for example, depicts a top view of the headset assembly 101 cooperated with the subject's head (not shown) using the stereotactical positioning via the registration system described above and with the temporal and occipital non-focused ultrasound beams 160, 162 emitted to target central cerebral vasculature 161 from two directions 161, 163. In this embodiment, the occipital transducer array 106 is shown to contain four occipital transducers 166. Fig. 9B is a "see through" side view showing how the ultrasound beams 162a, 162b of the occipital transducer array 106 are directed at the internal cerebral vasculature. When properly angled and positioned with the use the stereotactic registration system of an embodiment of the invention, as discussed above, the transducers in the occipital array are oriented towards the basilar and vertebral arteries as well as the junction of the internal carotids and anterior/posterior communicating arteries of the Circle of Willis 144.

*Examples of Auxiliary Elements and Operational Characteristics of Embodiments of the Invention.*

In one embodiment, and in further reference to Figs. 1, 2B, 7A-7D, for example, the transducer assemblies (105a, 105b, 106) are configured to be detachable from the headframe member 101a. In such embodiment, transducer arrays can be sold as disposable modules pre-fitted with a "ready-to-use" gel couplant pad. The transducer modules are optionally provided with wiring pins that are plugged into a female socket in the mounting receptacle (a portion of the headframe member 101a around the slide 154), thereby enabling an embodiment of the invention to perform a functional self-check with the use of an integrated watchdog circuitry before the beginning of insonation procedure. Optionally, the apparatus may also perform a self-check to verify acoustic coupling, such as by use of the phase comparator circuitry described later in this description, prior to initiation of cyclical metapulse emission.

Embodiments of the invention preferably employ non-focused transducers, i.e. transducers producing beams that diverge upon propagation and insonate broader target areas. For an unfocused transducer, the near field length, beam spread angle, and beam diameter can be calculated as $L = D^2 f c / 4c$, where $L$ is near field length, $D$ is element diameter or aperture, $f_c$ is the frequency, and c is the sound velocity in the medium.

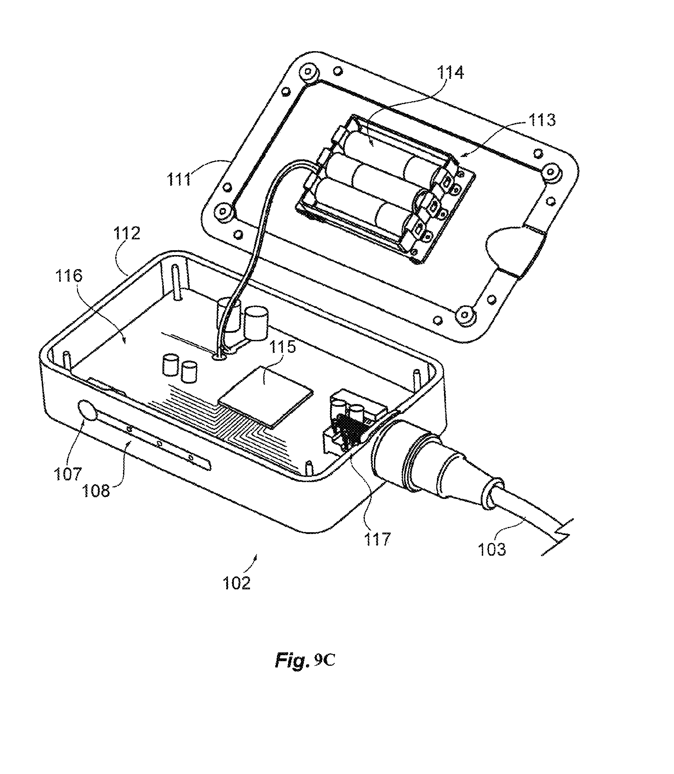

Fig. 9C is a perspective view illustrating the hand-held portable remote control unit 102 of Fig. 1 that has a housing 112 and a cover 111. As shown, the remote control unit 103 includes a printed circuit board 116 with a microcontroller 115 that is pre-programmed to enable an operation of the US device and optionally, to acquire and store data associated with such operation and/or insonation of the subject's head. For example, the remote control unit 102 optionally contains sensors (not shown) for collecting data and for generating feedback to the processor 115 to adjust at least some of the parameters of operation of the US device (such as output energy, sequence of firing of transducers or the pulse interval, for example). The remote control unit 103 also includes appropriate electronic circuitry (not shown) including tangible computer-readable storage medium. The electronic circuitry is configured to at least enable the operation of the transducers and, optionally, acquire data associated with such operation. Mounted at the cover 111 there is a battery pack power supply 113 (such as, for example, three AAA batteries 114). Also shown is an on-off/pause switch 107 and at least one status indicator 108 such as an LED-based indicator (or, alternatively, another appropriate status indicator such as a buzzer or a liquid crystal display). The printed circuit board 116 is supplied with leads to a junction 117 that forms a power and data bus routed through the umbilicus 103 to the headset 100 of Fig. 1. The power supply 113 may be equipped with a number of compact batteries commercially available that can deliver about 200 mAmp-hrs, 400 mAmp-hrs if needed, without recharging for up to about 12 hours at an operating voltage of about 1.5 to about 4 VDC, most preferably about 3.5 $\pm$ 1 VDC, but optionally about 9 - 12 VDC. The battery pack will thus have a capacity of 0.6 to 15 Watt-hours and preferably has a weight of less than 250 grams, more preferably less than about 100 grams, and most preferably less than about 50 grams. The battery may be generically a rechargeable battery, an insertable battery, a lithium ion battery, a lithium ion polymer battery, a lithium iron phosphate battery, a lithium-sulfur battery, a lithium-titanate battery, a nickel-zinc battery, a nickel-iron battery, a NiCd battery, a NiMH battery, an alkaline battery, a 9 V battery, a cell phone battery, or at least one AA or AAA battery (114, as shown), and the like. Preferably, the power supply is rechargeable and/or replaceable, and, where rechargeable, optionally includes a control circuit with a "fuel gauge" such as that available from Benchmark (BQ2040) for use in recharging the battery pack. Cell phone batteries are typically about 3.7 V and can deliver about 1 Amp-hr or about a specific power of 20 to 40 mAmp-hrs/gm or more. For example, an apparatus of the invention having a maximal power draw of 400 mAmp-hrs that is operated for 2 hrs in continuous mode and subsequently for 10 hrs in intermittent mode at 200 mAmp-hrs will require a battery pack of about 2.8 Amp-hrs capacity. An apparatus having a maximal power draw of 200 mAmp-hrs would require only 400 mAmp-hrs for operation over a 2 hr cycle, and hence could be operated with three AAA batteries in series supplying about 4 volts. New batteries could be installed if needed and the total weight of the battery pack is 50 gm or less, by way of example. Advantageously, this permits portable, extended operation, such as for ambulatory transcranial ultrasound.

In one embodiment, US transducers emit ultrasound in pulses that are optionally grouped in trains of pulses that, in turn, are modulatable in time and/or spatial position with the use of the processor 115. It was empirically determined that such modulation of the operation of the transducers reduces insonation intensity and power draw from the power supply while increasing efficiency of operation of an embodiment of the US device. An efficiency or rating factor for each transducer crystal stored in a tangible (and, optionally, computer-readable) memory unit associated with the US device may be accessed by the microcontroller 115 during startup of the insonation procedure and used to vary the voltage applied to each crystal of the headset independently from the operation of another crystal so as to compensate for variation in manufacturing of transducer arrays. This accomplishes an advantageous reduction in intra-headset variability of ultrasonic treatment.

Frequency, pulse repetition pattern, and pulse metacycle rate are among the factors in efficacy and safety of an US device according to an embodiment of the invention. Consequently, regimes of operation of the transducers and the corresponding patterned waveforms can be varied with the use of the pre-programmed processor 115. Primary frequencies selected for operation of the devices of the invention are in the range of about 0.5 to about 3.5 MHz. As the operational frequency is increased, a mechanical index (MI) of the insonated tissue decreases, but a thermal index (TI) of the insonated tissue increases inversely. (The MI is an indicator of the likelihood of non-thermal bioeffects in the tissue and is defined as the peak rarefactional pressure or derated peak pressure at negative amplitude, divided by the square

$$MI = \frac{P_{r.3}}{\sqrt{f}}$$

root of the frequency of the ultrasound, As the MI increases, the likelihood of bioeffects within tissue increases. Regulatory limits generally allow a mechanical index of up to 1.9 to be used for most tissues except ophthalmic. At low acoustic power, the acoustic response is generally linear. The TI is a calculated estimate of temperature increase with tissue absorption of ultrasound, determined by the ratio of the total acoustic power to the acoustic power required to raise the tissue temperature by 1°C. Some devices further subcategorize the TI according to the insonated tissue: soft tissue thermal index TIS for soft homogeneous tissues, cranial bone thermal index TIC for bone at or near the surface, and bone thermal index TIB for bone after the beam has passed through soft tissue. More generally, the temperature of insonated tissue increases with increasing intensity and with increasing frequency.) A range of operational frequencies from about 0.8 or 0.9 MHz to about 3.0 MHz is preferred. More preferable are the frequencies at about 0.8 MHz and about 1.2 MHz. In operation, pulse width, intensity and pulse repetition frequency are chosen such as not to exceed an integrated $I_{spta.3}$ limit of about 720 mW/cm$^2$. (Conventionally, a spatial-peak temporal average intensity, or $I_{spta}$, represents a value of the temporal average intensity at the point in the acoustic field where the intensity is at a

maximum. $I_{spta\cdot 0}$, for example, is calculated as $I_{spta,0} = (\varrho c^{-1}) * \int_{0}^{PD} P_r^2(t)/dt$, where PD is the pulse duration and $P_r$ is the peak rarefaction pressure defined as the absolute value of the half-amplitude of a sound pressure wave passing through the tissue.) Control of acoustic pressure and/or intensity is achieved by adjusting the voltage applied to a given transducer ($V_{p-p}$).

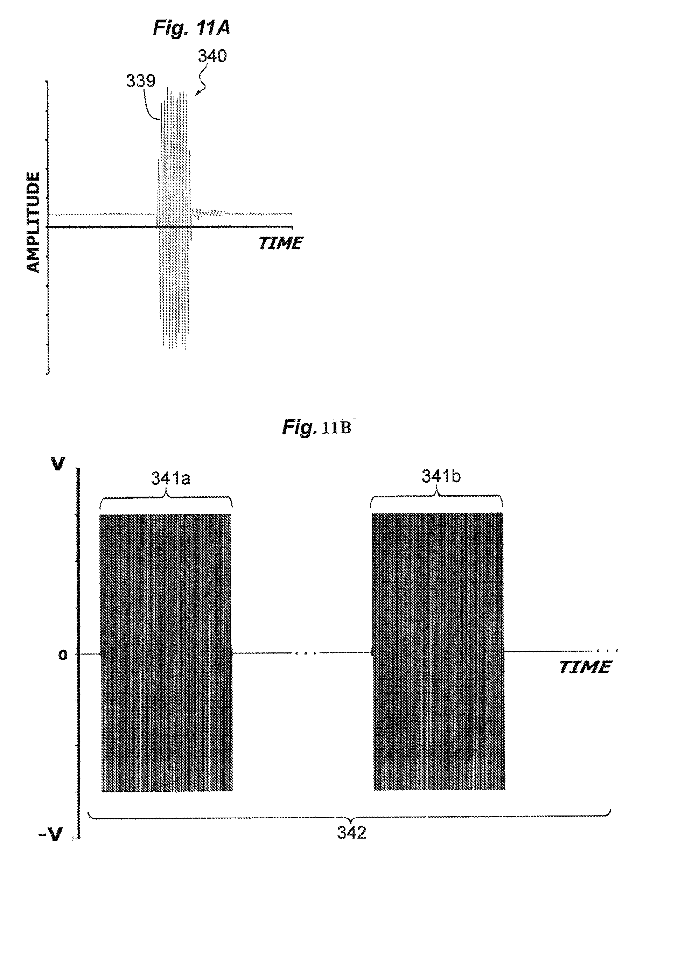

In defining regimes of operation of transducers of an US device according to embodiments of the invention, the processor 115 of the remote control unit 102 is optionally pre-programmed to cause emission of US in a form of various pulses (including a metapulse having cyclical metapulse repetition frequency, or MCRF) and duty cycles to limit power consumption and permit heat dissipation by passive means such conductive and convective cooling from external surfaces of the headset and transducers, and are configured not to overstress the cooling capacity of the wearer. MCRF is the frequency at which complete pulse cycles are emitted from a plurality of transducers of a headset such that each transducer emits ultrasound independently and in isolation from other transducers, in a predetermined sequence. Eliminating the need for active cooling dramatically decreases overall power draw of the apparatus and is an advance in the art. Fig. 11A, for example, illustrates a typical US pulse 340 formed by sinusoidal sound waves 339 at a primary frequency $f_c$. Each pulse 340 includes about 12 sound waves. At the primary frequency of 2 MHz, for example, a pulse of this kind has a pulse width of about 6 microseconds. As an example, a series of pulses 340 can be emitted by transducers of the US device of the invention with a pulse repetition frequency, or PRF, of about 6 KHz, which corresponds to a pulse interval, PI, of about 167 microseconds and a duty cycle of about 3.6%, while not limited thereto. Duty cycle may range from 0.1 - 10%, more preferably 3 - 5%, and most preferably about 3.5±0.5%. In other embodiments, pulse amplitude modulation or pulse frequency modulation may also be used. The PI value is selected so that the time between consecutive pulses or bursts from transducers of an array is generally no shorter than the time of flight of a pulse, including the return echo from the contralateral wall of the skull. In one embodiment, pulse intensity is such that the return echo is generally dissipated before the next pulse is emitted. This may correspond to a pulse interval of about 167 microseconds. Pulse interval is thus in the range of 150 to 300 microseconds, more preferably 170 to 250 microseconds. In this way, the pulse interval is configured to prevent echoes from constructively interfering with outgoing pulses and increasing local rarefaction pressures beyond an acceptable limit of about 300 KPa. Synergically, this configuration achieves lower power consumption and permits extended operation where portability is needed.

Fig. 11B illustrates, not to scale, a pair of pulse trains 341a, 341b each of which includes multiple pulses. For illustration, 100 to 300 pulses may be grouped in a single pulse train fired from a single transducer. A repeated cycle of consecutively fired pulse trains constitutes a "metapulse" 342. In one embodiment, simultaneous firing of particular transducer pairs or triplets may be used where amplitude is not additive.

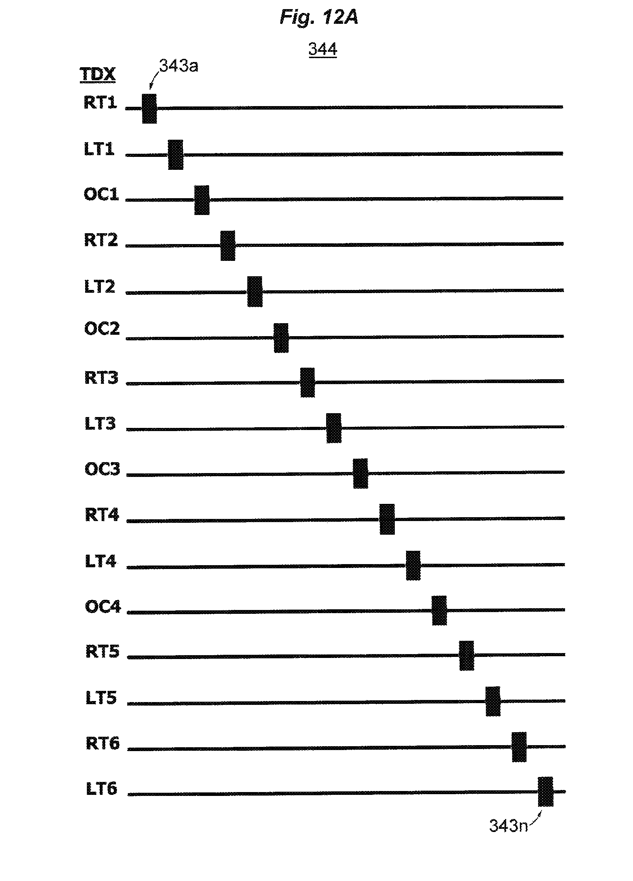

The overall pattern or waveform of US emitted by an embodiment of the invention optionally includes a cyclical pattern of spatially distributed and temporally modulated metapulses formed from sub-patterns of pulse trains and constitutes a regimen. Turning to Fig. 12A, for example, another regime of operation of an embodiment of the US device is shown, this time defined by a metapulse 344 having MCRF of about, for example, 2 Hz. Along the left margin, individual traces are labeled with designations for particular transducers of each array, for example "RT1" is right temporal #1 transducer, "OC1" is occipital #1 transducer, and so forth. The figure represents one complete firing sequence of the headset, one cyclical "metapulse" 344, which is emitted repeatedly. One pulse train is fired from each of sixteen transducers in the course of a single metapulse. Thus the timeline can be viewed as a staggered chronology where a first transducer emits a first pulse train 343a, a second transducer emits a second pulse train, and so forth, until the last transducer emits the last pulse train 343n concluding the pulse-train cycle. Accordingly, by appropriately defining a sequence according to which transducers from different transducer arrays are actuated, the formed pulse trains strike the target anatomical structure like an "acoustic nudge", each acoustic perturbation arriving from a somewhat differing direction as the metapulse cycles. A 6 KHz pulse train of 100 pulses has a duration of about 16 milliseconds, and could be termed a "nudge" for stimulating streaming. (Streaming or microstreaming are the terms used in the art to denote an effect of ultrasound on the behavior of insonated liquids, including increase in enzymatic fibrinolysis with low-intensity ultrasound include, increase of plasminogen activator binding to fibrin and transport into the clot. Acoustic streaming and microstreaming also promotes flow of interstitial and blood fluids, as described for example in U.S. Patent 3,961,140). The cyclical repeated metapulse is thus a "super-nudge" composed of multiple "nudges," each nudge a train of pulses. Fig. 12A illustrates, therefore, a patterned cyclical emission or "metapulse" of pulse train emissions from an array of sixteen crystals, where emission of each crystal emission is uniquely directed and is fired once per cycle. It can be said that the insonation regime is a cyclically repeating metapulse comprising one or more patterns of temporally modulated and spatially distributed pulse trains, each pulse train comprising multiple ultrasonic pulses. The insonation can thus be characterized as cyclically and stereotemporally modulated insonation. While not limiting in characterizing the invention, a headset with 16 transducers firing 16 millisecond pulse trains, one at a time, will cycle sequentially about every half a second. Of course, other permutations and/or sequences of operation of the employed transducers to form a specific pattern of temporally modulated and spatially distributed acoustic beams are also within the scope of the present invention.

In general, patterned waveforms of programmed insonation comprise a train of pulses, each said pulses having a pulse duration of about 0.2 to 10 microseconds (in certain embodiments more preferably about 1 to 8 microseconds, and in related embodiments most preferably about 6 microseconds), in trains of pulses of 2 to 300 pulses per train (in certain embodiments more preferably of about 100 to about 300 pulses per train), said train of pulses having a pulse repetition frequency of about 3 KHz to about 10 KHz, (in related embodiments more preferably about 4 KHz to about 8 KHz, and most preferably about 6 KHz), with an amplitude measured as unattenuated peak rarefaction pressure $P_{r0}$ of about 0.3 to about 1.0 MPa or more, and at a ultrasonic frequency of 0.5 to 3.5 MHz (and, in some embodiments, more preferably about 0.8 or 0.9 to about 3.0 MHz, and most preferably about 1 MHz, or about 1.2 MHz, or about 2.0 MHz). The pulse trains are also vectored from multiple independently fired transducers, thus resulting in spatial modulation or distribution of the patterned waveforms.

An apparatus of the invention has program instructions stored on tangible computer-readable non-transitory medium (discussed in reference to the processor 115 of Fig. 9C) that encode for autonomously driving a plurality of ultrasound transducers to emit a cyclically repeating metapulse, the metapulse comprising a wavepattern of spatially and temporally modulated pulse trains of ultrasound having a primary frequency $f_c$, and an amplitude configured to achieve a $P_{rAzsp}$ not to exceed 300 KPa, the pulse trains having a pulse repetition frequency corresponding to a duty cycle of 1 - 10%, more preferably 2 - 6%, and most preferably about 3 to 5% per transducer, the metapulse having a metapulse cycle repetition frequency of 0.25 to 10 Hz, until a stop instruction is received; thereby achieving low power consumption for extended portable operation independent of operator control and not requiring assisted cooling means.

In a preferred embodiment, the individual transducers may be about 1 cm in diameter or smaller and are spaced apart (unlike conventional phased array transducer assemblies) to permit heat dissipation between firings. The transducer crystals are not fired in pairs and the beams emitted are not convergent, but are instead fired individually in series under autonomous control of a remote controller unit, which contains a clock, pulse generator, logic circuits for transducer actuation, and optionally with control of amplitude of individual transducer output as compensation for attenuation or inter-transducer variability. This multiple mini-transducer approach has been proven to be safe, cognizant of the dangers of standing waves and heating, and is found to be surprisingly effective in sonothrombolysis. While not bound by theory, the device is thought to stimulate diffusion of r-tPA by insonation-induced fluid streaming in response to modulated pulsed ultrasound patterns directed at a target anatomy from multiple directions, where a first transducer is fired with a pulse train or "acoustic nudge", a second transducer is then activated, and between each pulse of each pulse train, the emitted ultrasonic wavefront is allowed to fade in intensity. Taking into account a temporal-temporal or occipital-frontal beam path length of 10 to 15 cm in a typical application, the pulse interval is thus on the order of 150 to 300 microseconds, as was described in more detail in the preceding section.

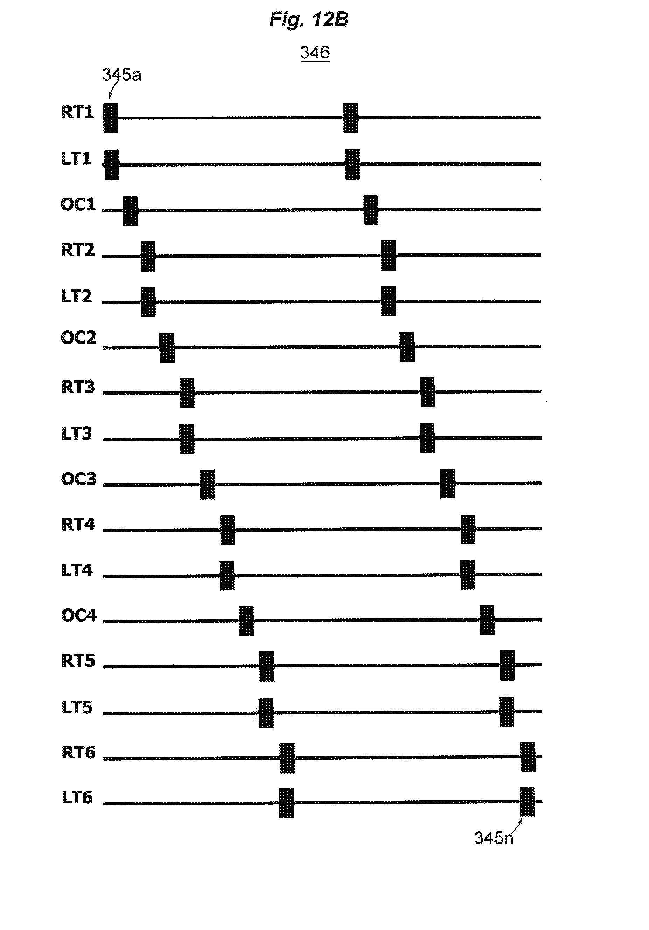

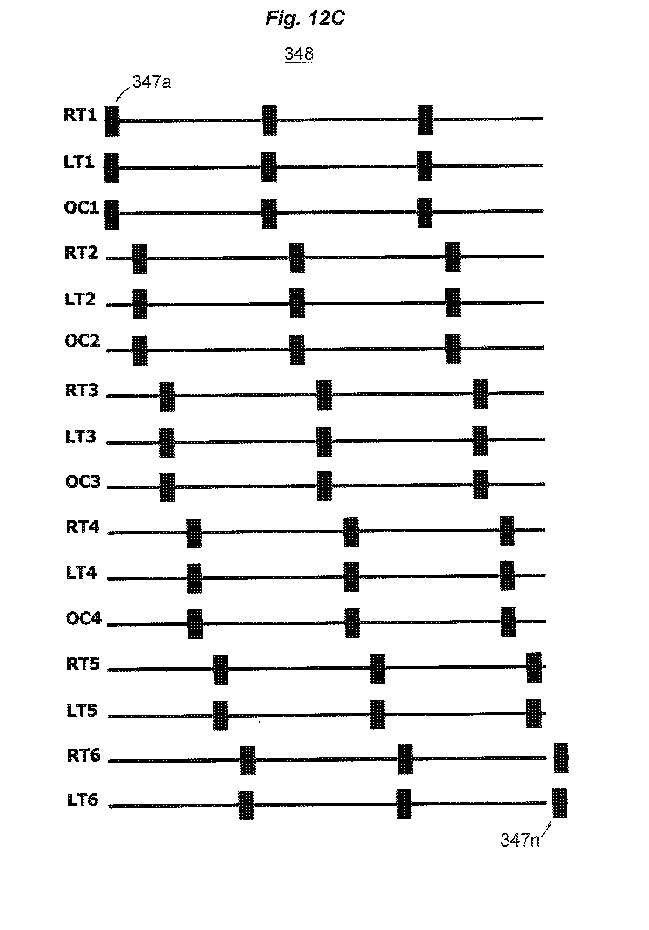

Other pulse and metapulse sequences, defining a pre-programmed regime of operation of transducers of an embodiment, are also within the scope of the invention. Fig. 12B, for example, illustrates describes a metapulse sequence 346 where two metapulses are shown (repeating doublet pattern, left to right). Each metapulse includes pulse trains fired by various pairs of transducers simultaneously. The doublet pairs selected for simultaneous firing are generally contralateral pairs so as to minimize potential additivity in the amplitude of the peak pressures where the beams meet. Study has shown that contralateral beams can be configured to overlap so that constructive addition of beam intensity is beneficial at target depths of 4 to 7 cm (for each hemisphere) and does not exceed safe limits at any depth. In this instance, contralateral doublet pulses are emitted simultaneously but the pulse interval between consecutive doublet pairs is on the order of 150 to 300 microseconds. Contralateral doublet pulses are particularly useful at higher frequencies, such as at 2 MHz, where pulse attenuation is more rapid. The example of Fig. 12B shows 32 emitted pulse trains (345a - 345n), but generally the number of pulse trains may be different. Fig. 12C describes a metapulse sequence 348 consisting of triplets and doublets, where three metapulses are shown (repeating pattern, left to right). Each doublet or triplet consists of simultaneous pulse train emissions by two or three transducers, respectively. Again these are chosen so that additive effects are minimized but are most beneficial at depth. By firing three transducers at once, where the individual transducers are selected from the right temporal, left temporal and occipital arrays, amplitudes do not rise above safe levels where beams overlap. The example of Fig. 12C shows the total of 48 pulse trains emitted by the transducers.

In a specific embodiment, the apparatus of the invention is configured to operate by emitting ultrasound in stereotemporal metapulses, each metapulse including a series of pulse trains emitted in a pattern such as, for example, a) a series of pulse trains emitted in sequence from a plurality of transducers, wherein only a single transducer is actuated according to a programmed order at any given time; b) a series of pulse trains emitted from pairs of transducers selected from a plurality of transducers, wherein only one pair of transducers is actuated according to a programmed order at any given time; c) a series of pulse trains emitted from triplets of transducers selected from a plurality of transducers, wherein only one triplet of transducers is actuated according to a programmed order at any given time; d) a series of pulse trains formed by actuating transducers in a sequential order, the series comprising any combination of singlet, doublet, or triplet transducer actuations; or, e) a series of pulse trains emitted on a carrier wave, the carrier wave having a sub-ultrasonic frequency of about 5 to 10 KHz, and most preferably about 6 KHz.

At a clinician's discretion, individual arrays or transducers may be actuated more frequently than others, for example, when it is desirable to preferably insonate a particular hemisphere of the brain or a frontal versus an occipital aspect of the cerebral vasculature. In other instances, particular transducers are chosen to fire more frequently than others so as to optimize acoustic streaming in a particular direction, such as circularly in the Circle of Willis by firing posteriorly-directed Rt temporal transducers in alternation with anteriorly-directed Lt temporal transducers in alternation, and then reversing the direction by firing anteriorly-directed Rt temporal transducers in alternation with posteriorly-directed Lt temporal transducers, so as to create clockwise and counterclockwise pressure gradients which stimulate directed acoustic streaming and flow. Also of interest are reciprocating pressure pulses, such as alternating pulse trains between matching transducers situated contralaterally in the temporal arrays, or orthogonally directed pulses in alternation from ipsilateral transducers of the temporal and occipital arrays, for example.

By firing only a few transducers at a time, and by firing individual transducers (as determined by the pulse repetition frequency) at a duty cycle in the 1 to 10% range, more preferably in the about 3 to 6% range, and in one embodiment with a duty cycle of about 3.6%, the need for assisted cooling is avoided. TI and thermal heating effects due to longer duty cycles are limited. This approach permits use of higher frequencies, which can be advantageous because mechanical index (MI) is more easily limited. Lower power consumption also results; without loss of efficacy. The apparatus is typically passively air-cooled, avoiding power consumption by fans, circulating coolant, and so forth.

The requisite pulse interval can be achieved with a pulse repetition frequency of about 4 to 10 KHz, more preferably about 5 to 8 KHz, and most preferably about 6 KHz. Fortuitously, this PRF is more physiologically compatible than lower frequencies in that users have been observed to perceive pulsed insonation in the 2 - 4 KHz range, in particular, as an uncomfortable sound; paradoxically sensing, by a sort of biological demodulation, what is by definition an inaudible ultrasonic pulse. The 0.5 to 3.5 MHz primary frequency is well above the range of human hearing but can be "demodulated" when pulsed at 2 - 4 KHz.

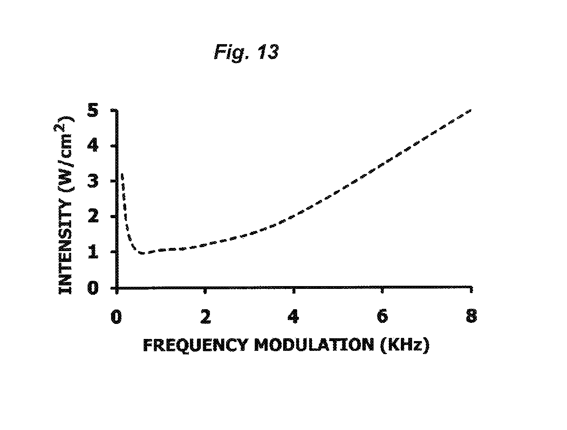

Fig. 13 quantifies the threshold for audible sensation of ultrasound exposure as a function of a modulated pulse train frequency. Greater amplitudes are required to elicit a sensation outside the range of 0.2 to 4 KHz. Pulse repetition at frequencies greater than about 4 KHz are less likely to be perceived by the user. Frequencies in the 2 kHz to 3 KHz range are most perceivable. It is empirically determined that a selection of a 5 kHz or a 6 KHz PRF is most appropriate for perception by most individuals and, at a duty cycle of 3 to 6% or so, poses no significant risk of excessive heating or overexposure as measured by TI and $I_{sppa.3}$. At PRF greater than 10 KHz, a spatial overlap of successive wave patterns can be associated with standing waves, so RPF of about 4 kHz to about 10 KHz has proved to contain a narrow range of comfort where safety of the subject is not compromised.

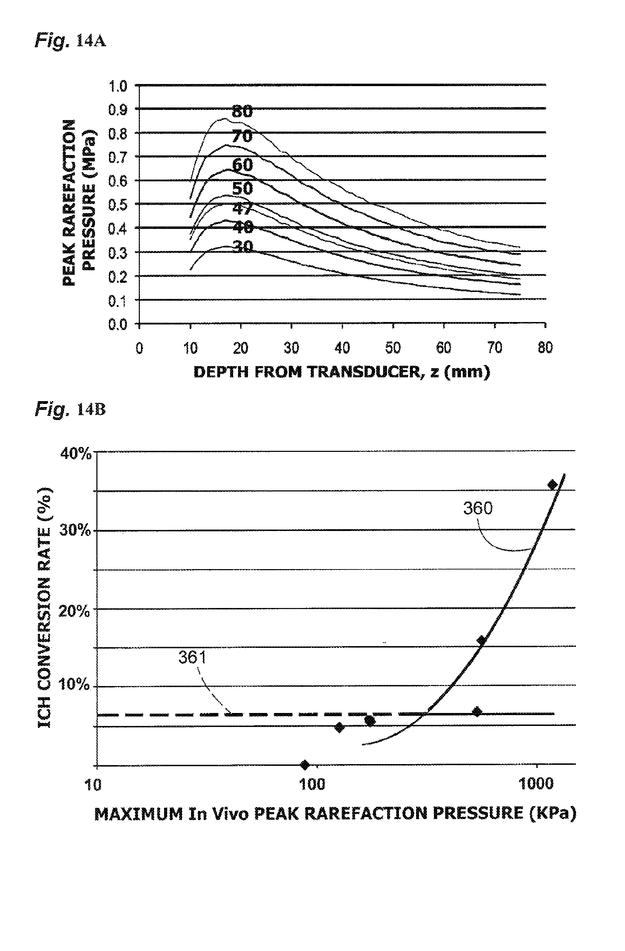

Insonation amplitude is directly related to peak rarefaction pressure, and the circuitry is configured to deliver no more than 300 KPa at a depth of $z_{sp}$. Fig. 14A shows peak rarefaction pressure as a function of depth for selected increments in transducer voltage ($V_{p-p}$) from 30 to 80 $V_{p-p}$. Fig. 14B plots the relationship between peak rarefaction pressure (KPa) and intracranial hemolysis (ICH) conversion (%) as determined here from retrospective analysis of data from clinical trials. The localized acoustic intracranial pressure, as correlated with maximal peak rarefaction pressure 360, is believed to be responsible, at least in part, for the increase in ICH over untreated baseline (361) shown in Fig. 14B. The relationship between applied acoustic pressure at the transducer face and resultant acoustic pressure at a depth in the skull is dependent on a complex analysis of attenuation. It can be seen that 300 KPa represents a threshold where increases in insonation amplitude are associated with increases in ICH conversion, a technological problem not previously recognized. Based on this analysis, a specific embodiment of the device configured to operate such as not to exceed the $P_r \leq 300$ KPa threshold in target tissues was evaluated. Coincidentally, this configuration operates for longer periods of time at lower power, and is found here to improve therapeutic outcomes, a technological advance in the art.

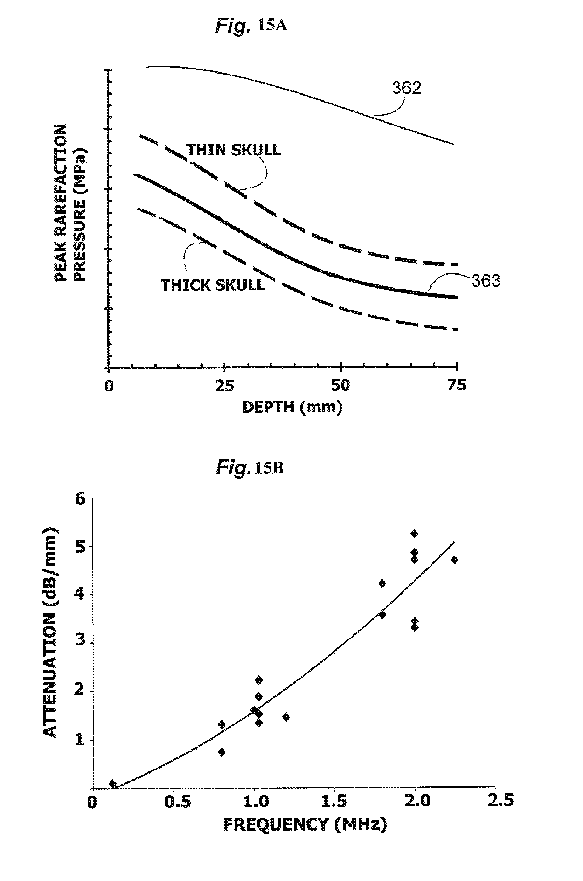

In a first example demonstrating the role of attenuation, Fig. 15A is a plot of peak rarefaction pressure 363 illustrating the effect of thick and thin skull phenotype as a function of depth at 1 MHz. Also shown is a curve for unattenuated emission 362. Fig. 15B is a curve fit for attenuation coefficient ($A_{TempBONE}$) versus frequency for temporal bone. Attenuation coefficient $\alpha$ is given in dB/mm-MHz.

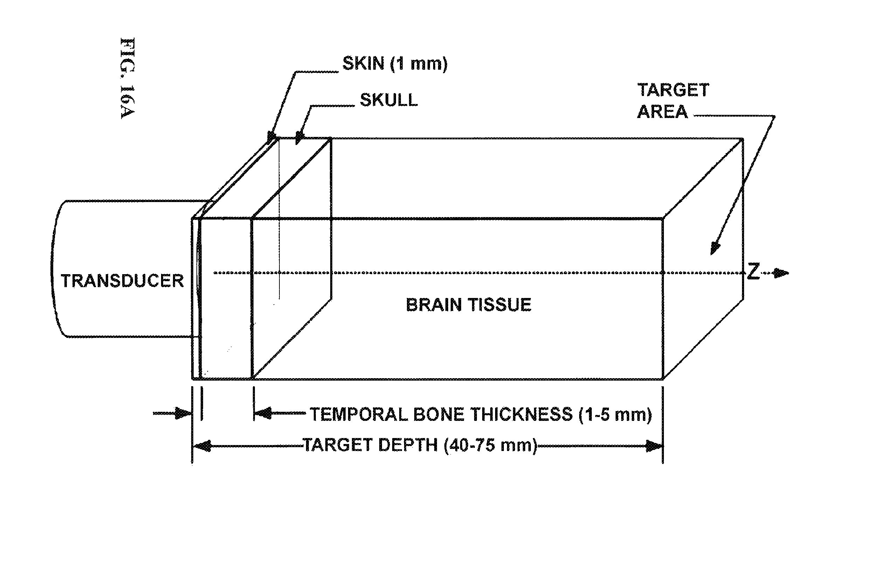

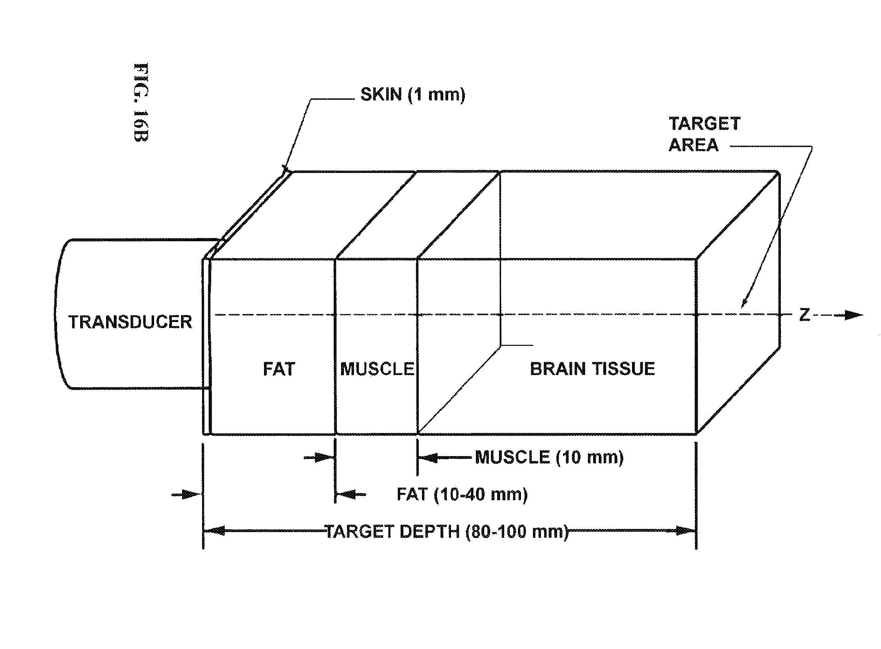

Figs. 16A and 16B present physical models for analysis of an attenuation profile as a function of depth for a transducer seated at a temporal acoustic window (Fig. 16A) and for a transducer apposing an occipital acoustic window (Fig. 16B), where a layer of fatty tissue generally covers the muscles the spine that overlie the atlas and foramen magnum. The outlines of a calculation of attenuation are first shown pictographically. The renderings represent physical models for analysis of an attenuation profile as a function of depth for a transducer seated at a temporal acoustic window (Fig. 16A) and for a transducer apposing an occipital acoustic window (Fig. 16B), where a layer of fatty generally covers the muscles the spine that overlie the atlas and foramen magnum.

*Related Mathematical Description.*

Developing a mathematical description of the temporal interface model (corresponding to Fig. 16A) requires information about sound velocities and attenuation in the various tissue types. Tissue-specific attenuation constants can be approximated from literature values factored for measured thickness and the combined attenuation profile can then be calculated as a function of depth, where attenuation of $P_r$ across a temporal acoustic window is given as

$$|P_r(Z)| = \frac{P_{rAz}}{P_{rUz}}$$ (1)

where $P_r(Z)$ is the ratio of attenuated ($P_{rAz}$) to unattenuated ($P_{rUz}$) ultrasonic pressure as a function of depth (z) in centimeters. The attenuation parameter can be determined from

$$A_{TOTAL} = A_{SKIN} + A_{BONE} + A_{REFLECTION} + A_{BRAIN}$$ (2)

and

$$A_{TOTAL} = 20 \cdot \log_{10} \frac{P_{rAz}}{P_{rUz}}$$ (3),

to yield

$$P_{rAz} = P_{rUz} * 10^{\frac{(A_{SKIN}) + (A_{BONE}) + (A_{REFLECTION}) + (A_{BRAIN})}{20}}$$ (4)

where is the attenuated peak rarefaction pressure at depth z on the beam path; is the unattenuated peak rarefaction pressure at depth z; $A_{SKIN}$ is the acoustic attenuation in the outer skin and tissue; $A_{BONE}$ is the acoustic attenuation through the skull bone; $A_{REFLECTION}$ is the attenuation equivalent to reflection losses at the interface between the inner surface of the skull and the brain, nominally 3.02 dB; $A_{BRAIN}$ is an acoustic attenuation in the brain, typically a constant at about 0.06 dBhnm-MHz; z is a depth, the distance a ultrasonic beam wavefront has traversed; $t_{SKIN}$ is the thickness of the outer skin and tissue layer; $t_{SKULL}$ is the thickness of the cranial bone proximate to the transducer; and, $f_c$ is the center frequency (MHz).

Each component is now considered separately. The following attenuation coefficients, taken from the general literature, are tabulated for reference in Table 1:

Table 1.

| Tissue Type | $\alpha_{TT}$ (dBhnm-MHz) |
|---|---|
| Skin | 0.18 |
| Fat | 0.04 |
| Muscle | 0.09 |
| Sub-occipital brain tissue | 0.09 |
| Temporal brain tissue | 0.08 |

Attenuation introduced by skin is typically small and may be neglected, but is given by

$$A_{SKIN} = \alpha(\sigma_{SKIN} \cdot f_c) \cdot t_{SKIN}$$ (5).

In comparison, bone attenuation is significant and is dependent on thickness of the bone in the path of the transducer beam, frequency and is best described by a non-linear curve fit of physiological data. The attenuation for temporal bone ($A_{TempBONE}$) was derived by parabolic curve fit of available data (Fig. 15B) and is described mathematically as

$$A_{TEMPBONE} = (-0.186 \cdot f_c^2 + 3.257 \cdot f_c - 1.51) * t_{SKULL},\qquad (6)$$

The regression fit ($R^2$) for data acquired with the use of Eq.(6) was 0.875. Brain attenuation is given by

$$A_{BRAIN} = \alpha_{BRAIN} \cdot t_{BRAIN} \qquad (7)$$

Reflection attenuation at the skull/brane interface is a function of the relative difference in acoustic impedance between the temporal bone and underlying brain tissue and is essentially a constant:

$A_{REFLECTION}$ = 3.02 *dB* Knowing boney layer thickness and having reference values for constant terms, the total attenuation plus reflection ($K_{SKULL}$) in decibels is readily determined. A similar measurement and calculation may be made for skin. In most instances, the attenuation associated with the outer skin layers is negligible compared to the larger contribution of the cranial bones, so equation 3 is further simplified to:

$$P_{rAz} = P_{rUz} * K_{SKULL} * 10^{\frac{A_{BRAIN} \cdot (z - (t_{SKIN} + t_{SKULL})) \cdot f_c}{20}} \qquad (8)$$

where $P_{rAz}$ is the calculated pressure at depth z after tissue attenuation and $P_{rUz}$ is the measured rarefaction pressure at depth z as measured in a tank of water. Eq. (8) is readily solved by a calculating machine, such as a microcontroller with on-board math functionality when given skull thickness, and permits the device to predict $P_{rAZsp}$ (peak rarefaction pressure at $z_{sp}$) as a function of depth based on a measurement of skull layer thickness such as by CT scan.

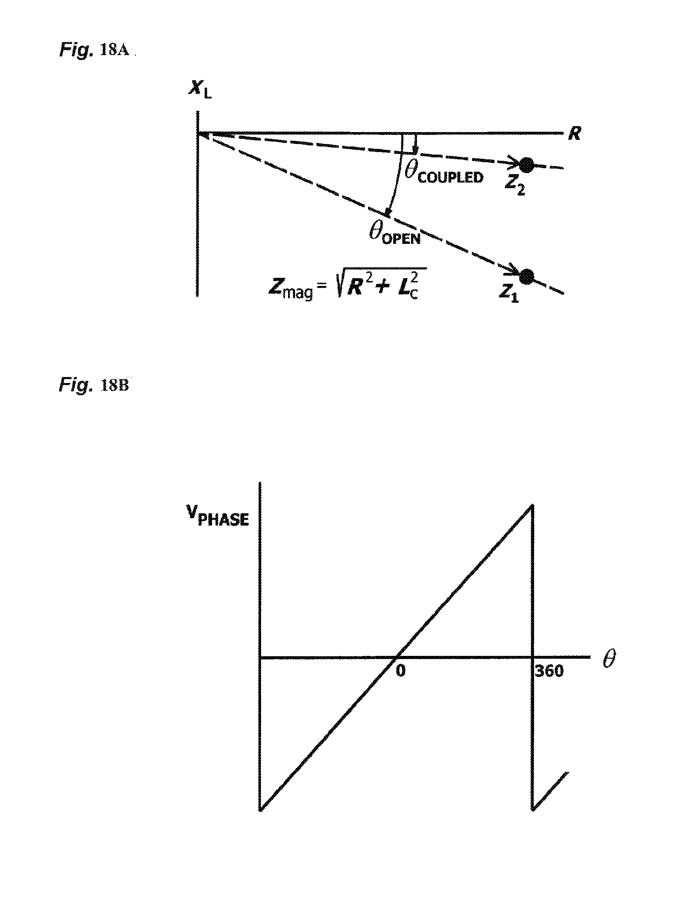

Turning now to Fig. 16B, a physical model for analysis of an attenuation profile as a function of depth for a transducer seated at a sub-occipital acoustic window is described. Fig. 18B depicts a sub-occipital transmission path for ultrasonic energy into the cranial vault between the spinal column and the foramen magnum. The physiology corresponding to the sub-occipital acoustic window is distinguished from that of the temporal acoustic window by a general absence of a boney layer. Moreover, attenuation of US associated with the US propagation through the sub-occipital acoustic window is substantially smaller that that corresponding to the temporal acoustic window. It is assumed that the primary mode of wave transmission is transverse, i.e. wavefront associated with the emitted waves is substantially parallel to the emitting facet of a corresponding transducer. As the ultrasound pressure wave propagates from the transducer to the point of maximum exposure of the cranium, the overall model for attenuation along the sub-occipital path can be approximated by evaluating the transmission characteristics across four major layers each with a tissue attenuation coefficient (skin, fat, muscle, and brain tissue as shown in Fig. 16B.

The attenuation parameter can be solved from

$$A_{TOTAL} = A_{SKIN} + A_{FAT} + A_{MUSCLE} + A_{BRAIN} = 20 \cdot log_{10}\frac{P_{rAz}}{P_{rUz}} \qquad (9).$$

Accordingly,

$$P_{rAz} = P_{rUz} * 10^{\frac{(A_{SKIN}) + (A_{FAT}) + (A_{MUSCLE}) + (A_{BRAIN})}{20}} \qquad (10)$$

where $P_{rAz}$ is the attenuated peak rarefaction pressure at depth z on the beam path; $P_{rUz}$ is the unattenuated peak rarefaction pressure at depth z; $A_{SKIN}$ is the acoustic attenuation in the outer skin; $A_{FAT}$ is the acoustic attenuation through the bone; $A_{MUSCLE}$ is the attenuation through the fat layer; $A_{BRAIN}$ is an acoustic attenuation in the brain, typically a constant at about 0.06 dB/mm-MHz; z is a depth, the distance a ultrasonic beam wavefront has traversed; $t_{SKIN}$ is the thickness of the outer skin layer; $t_{FAT}$ is the thickness of the fat layer; $t_{MUSCLE}$ is the thickness of the muscle layer; and $f_c$ is the center frequency (MHz).

Each component is now considered separately. Attenuation coefficients ($\alpha$), were previously summarized in Table 1. Skin attenuation is typically small, but is given by

$$A_{SKIN} = \alpha(D_{SKIN} \cdot f_c) \cdot t_{SKIN} \qquad (11)$$

where thicknesses are on the order of 1 mm. Fat tissue attenuation is given by

$$A_{FAT} = \alpha(\square_{FAT} \cdot f_b) \cdot t_{FAT} \qquad (12)$$

Based on empirical observation of experienced TCD sonographers, the total tissue thickness on the back of the neck will vary between about 2 cm and about 5 cm, of which about 1 cm is muscle. Thus, the fat layer can vary between about 1 cm and about 4 cm.

Muscle tissue attenuation is given by

$$A_{MUSCLE} = \alpha(\square_{MUSCLE} \cdot f_b) \cdot t_{MUSCLE} \qquad (13)$$

Nominal muscle thickness is taken as 10 mm for most applications. Brain attenuation is given by

$$A_{BRAIN} = \alpha_{BRAIN} \cdot t_{BRAIN} \qquad (14)$$

Because of the large variability in the fat layer and the variability in the point of maximum pressure ($z_{sp}$) depending on transducer selection, it is possible that the actual point of maximum peak negative pressure could be in an adjacent layer to the brain instead of in the brain itself.

If the fat layer associated with the subject's skull is 1 cm thick, then the point of maximum peak negative pressure will certainly be inside the brain. Mathematically, if $z_{sp}$ = 3 cm, and the overlying layers of tissue are 2.1 cm thick (= 0.1 cm + 1 cm + 1 cm) (skin + fat + muscle), $P_{rAmax}$ is 0.9 cm into the brain tissue. $P_{rAmax}$ is the point of maximum acoustic amplitude in tissue. The computation of attenuation is straightforward unless $P_{rAmax}$ is situated in the connective tissue layers.

If, however, $Z_{sp}$ is inside the fat or muscle layer, the estimate changes. In this case it is necessary to consider the total attenuation across the skin, fat, and muscle and then estimate the peak negative pressure at the muscle-brain tissue interface. This will be the estimated maximum peak negative pressure ($P_{rAmax}$) in brain tissue.

For most ultrasound systems, the peak negative pressure is conservatively assumed to decrease by about 0.5% per centimeter. Thus, the maximum peak negative pressure in the brain tissue (before accounting for the other attenuation values) can be estimated (for $Z_{sp \leq} T_{SKIN} + T_{FAT} + T_{MUSCLE}$) from

$$P_{rAmax} = P_{rI(V\bar{z}_{sp})} \cdot \left(-0.05(t_{SKIN} + t_{FAT} + t_{MUSCLE}) + (0.05z_{sp}) + 1\right) \qquad (15)$$

And for zip > $t_{SKIN}$ + $t_{FAT}$ + $t_{MUSCLE}$,

$$P_{rAmax} = P_{rI(V\bar{z}_{sp})} \qquad (16)$$

These considerations and calculations are useful in selecting conditions for operation of headsets of the invention.

*Additional Configurations.*

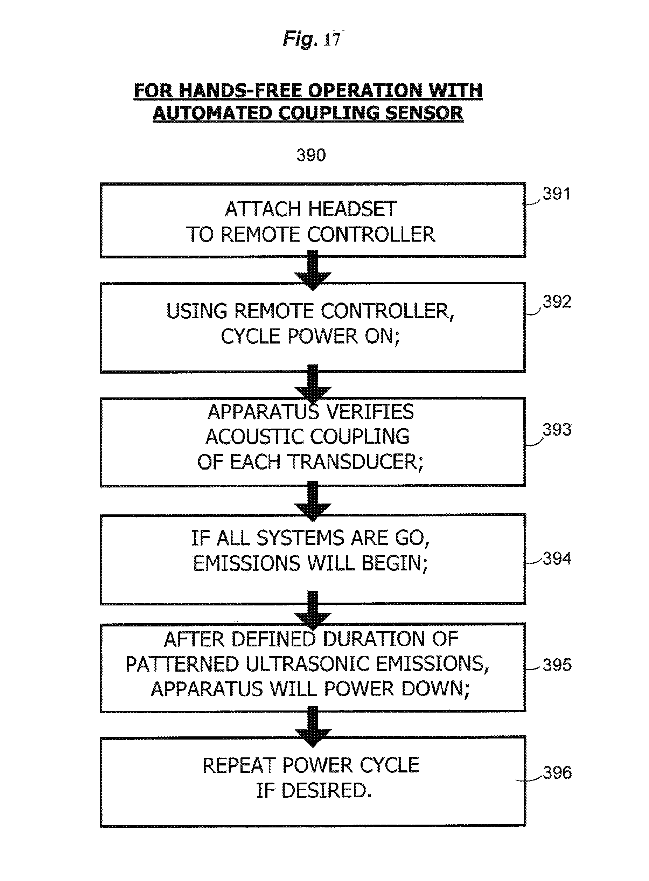

Fig. 17 is a flow chart or logic diagram for automated operation of an apparatus 390 where transducer coupling is tested and verified before or during operation of the apparatus. Following attachment of the headset to a skull at step 391, generally with gel on the transducers, the apparatus is powered on and performs a startup self diagnostic, at step 392. The processor of the apparatus then determines, at step 393, for each transducer whether the physical coupling with the head is sufficient for efficient transmission of sound. If all systems are GO and acoustic coupling is verified, the apparatus commences an insonation metapulse cycle, at step 394, and continues this until powered down at step 395 or for a duration of time preset in the programming. The cycle may be repeated if desired, as seen at396.

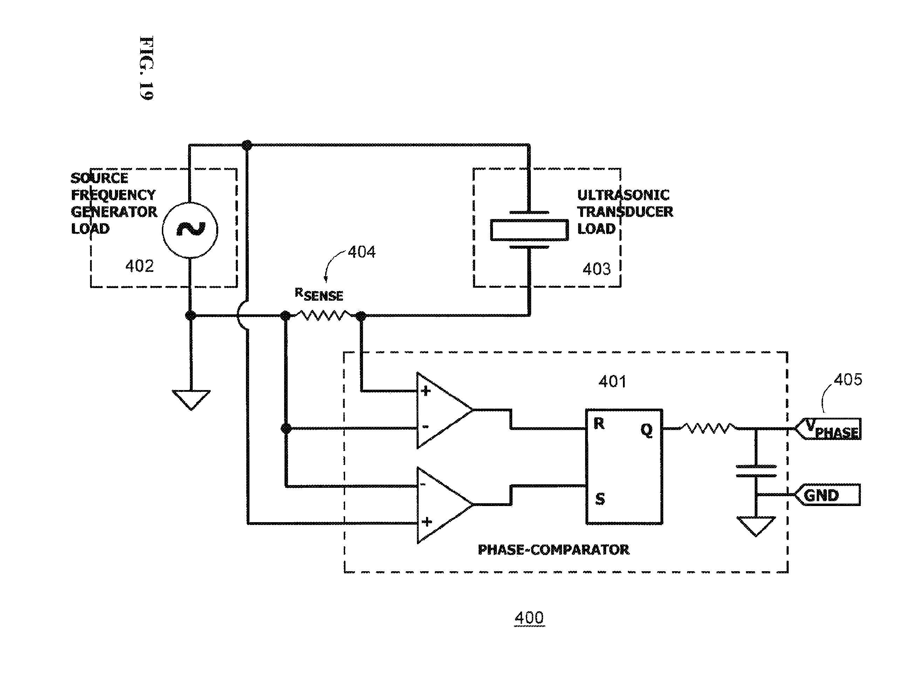

Figs. 18A and 18B describe the use of phase angle $\theta$ fro verification of the acoustic coupling. Fig. 18B plots voltage output corresponding to phase angle for a coupling verification circuit of Fig. 19. In this application, an ultrasound pulse may be used to validate acoustic coupling between the transducer interface and the underlying tissue target. If a transducer is not acoustically coupled to the underlying skull, generally with a couplant gel, the ultrasound bounces off the intervening air layer and fails to penetrate the skull. To verify coupling, in one embodiment a voltage comparator circuit is used to measure phase angle of the voltage pulse activating the transducer. A high phase angle is indicative of poor coupling, generally indicating the presence of air between the transducer and the target tissue. A low phase angle indicates good coupling. These circuits are practical solutions to the problem of ensuring good acoustic coupling before initiating an autonomous ultrasonic pulse treatment regime. The microcontroller will verify that the phase angle does not exceed a preset threshold before initiating insonation.

As shown in Fig. 18A, an impedance matching system may be designed to match source and load impedances when the headset is properly seated and acoustically coupled on the cranium. The signal source in this application is an amplified clock frequency emitted in pulses and the load is a piezoelectric transducer.

In considering an ultrasound transducer with a capacitive reactance, overall power load impedance is the sum of a real resistance "R" and an imaginary reactance "-jX". Current is shifted in phase by an angle $\theta$ relative to voltage. Total impedance $Z_{mag}$ is calculated as:

$$Z_{LOAD} = R - \frac{j}{\omega C} \qquad (17)$$

where R is resistance in ohms, $\omega$ is frequency expressed as radians, $\omega = 2\pi f$, where $f$ is the frequency in Hz, and C is capacitance expressed in Farads, which may also be written,

$$Z = \sqrt{R^2 + X_c^2} \qquad (18)$$

where $X_c$ is the capacitive reactance (ohm).

Taking the RC network and assigning the real part of the impedance to the real axis and the imaginary part to the imaginary axis, the impedance vector $Z_{mag}$ will appear as in Fig. 18A. The Pythagorean relationships for right angle triangle geometry, relating ordinate R, abscissa $X_c$ and hypotenuse Z, allows the impedance $Z_{mag}$ to be solved from the resistance and the capacitive reactance $X_c$. However, all that is needed to determine whether the surface of the transducer is acoustically coupled to an external load is the phase angle $\theta$. The phase angle ($\theta_{OPEN}$) will be large (ie. capacitive reactance will be large) when the transducer is acoustically mismatched with air, and will be dramatically lower ($\theta_{COUPLED}$) when the transducer is acoustically matched with the tissue of the skull. This observation is illustrated in Fig. 18A. Full measurement of the change in complex impedance (in phasor notation, $Z_{mag}\angle\theta$ for $Z_1, Z_2$) when coupling is established is not required for detection of an operative level of acoustic coupling between the crystals and the head of the subject wearing the headset. A rapid check of phase angle may be made by emitting an acoustic pulse and assessing phase angle of the pulse in a transducer. A circuit for digitally reporting the phase angle to a microcontroller is sufficient to assess acoustic coupling and the microcontroller can be programmed to perform this test individually for each transducer without operator intervention. If a transducer is not coupled, the operator will have to readjust the fit of the headset on the wearer, or eliminate any air between the transducer and the skin by adding gel couplant, for example.

An example of implementation of circuitry 400 for phase measurement on a transducer load 403 driven by an oscillator 402 is shown in Fig. 21. Here, such implementation is performed with an integrated circuit having a phase comparator, such as the HCT2046A (Philips Semiconductor, see 1997 Datasheet), which contains an edge triggered RS-type flip-flop phase comparator (PC3). The average output from phase comparator 401, fed to a voltage comparator via the low-pass filter and seen at the demodulator output at pin 10 (405, $V_{PHASE}$), is the result of the phase differences of $SIG_{IN}$ and $COMP_{IN}$ are generally linear between 0 and 360 degrees theta as shown in Fig. 18B. The output 404 from $R_{SENSE}$ can be offset to produce a desirable VPHASE = 0 at zero degrees. More details of the device are provided in the 74HC/HCT4046A Phase-Lock-Loop with VCO IC data sheet from Philips.

The realization of these considerations is a phase detection circuit with linear output voltage that can be digitally encoded to flag an uncoupled transducer in a headset array for corrective action, as is needed for use of the device by relatively unskilled technicians or for self-use. A simple LED may be used to indicate an uncoupled transducer, for example.

In autonomous operation of an apparatus configured for detection of acoustic coupling under each transducer prior to initiation of insonation, the apparatus will fault if coupling requires adjustment.

A variety of watchdog circuits to verify proper function before initiating insonation may be employed. Status lights or other indicator such as sounds, buzzers, LEDS, or even a liquid crystal display may be used to communicate the readiness of the device to begin ultrasound emissions. The LCD may for example scroll a message indicating that one of the transducers is not properly seated on the head. Status displays may also include battery status indicators, temperature sensors and indicators, and the like. Circuit fault detectors within the skill of those who practice electronic arts may also be incorporated.

Generally, frequency is also known, or easily measured, permitting use of $Z_{mag}\angle\theta$ information in other calculations, such as time of flight, which may be utilized in rudimentary imaging of midline shift conditions and quantitation of total dosage (from measurement of transducer-to-transducer pulse reception).

Earlier reports indicated partial success with highly focused ultrasound, where individual clots were visualized using TCCD and then insonated at 2 MHz at about 400 mW/cm$^2$ (Cintas et al, 2002, High Rate of Recanalization of Middle Cerebral Artery Occlusion During 2-MHz Transcranial Color-Coded Doppler Continuous Monitoring Without Thrombolytic Drug, Stroke 33;626-628). This study required precise sonographic positioning of the transducer and focused insonation at the precise site of the clot.

Surprisingly however, the use of unfocused transcranial ultrasound, modulated temporally and spatially as described in this application, is provide useful without exogenous administration of r-tPA. Modulated ultrasound is directed at the cerebral vasculature as a whole from multiple directions (i.e. spatially distributed modulation) in a series of patterned pulse trains, what is essentially a pattern within a pattern within a pattern, where pulse trains of pulses are emitted from individual non-focused transducers and a plurality of transducers of a plurality of arrays are fired in a patterned sequential order (i.e., as a metapulse, which are spatially and temporally patterned pulse trains of pulses). Ultrasound may be provided in this way without specific information about the presence or location of a clot and may also be performed prophylactically without a diagnosis if desired.

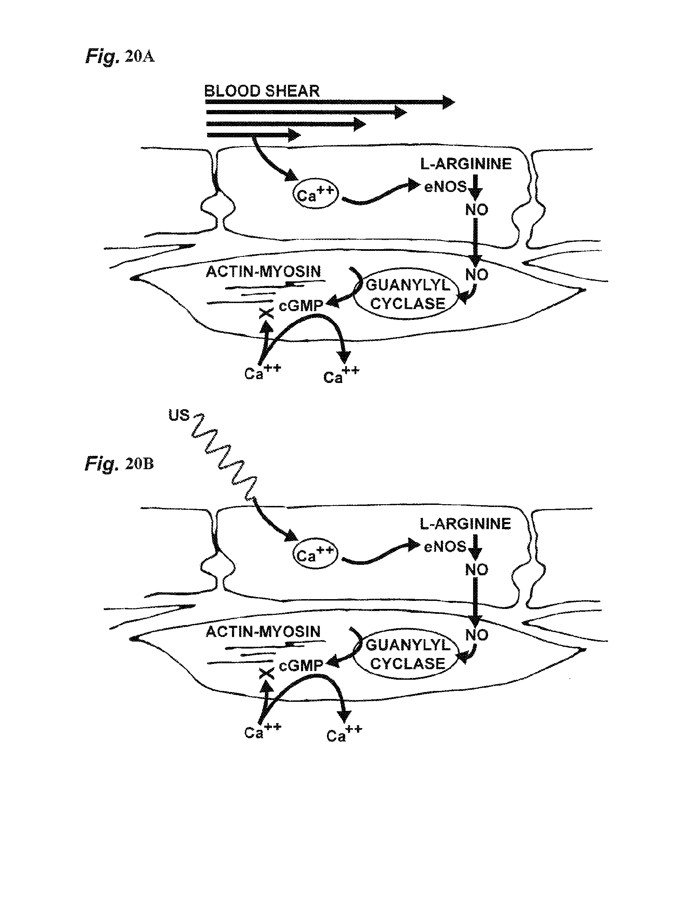

Figs. 20A and 20B depict a model for vascular vasodilation with release of endogenous nitric oxide, where blood shear (Fig. 20A) is replaced by ultrasound (Fig. 20B). This model predicts that the ultrasound device of the present invention will have a positive effect as a standalone apparatus for non-invasive treatment of a variety of conditions, including migraine, headache, intracranial hypertension, hydrocephalus, and so forth, and may increase blood flow to the brain so as to improve delivery of a variety of parenteral and oral drug formulations.

The structural members of the headset of the device are preferably built entirely from plastic and elements of electronic are isolated from the headset so that the headset is operated under remote control, thereby permitting insertion of the headset into a CT or MRI machine for CT/MRI scanning

*Industrial Applicability and Examples of Practical Use.*

In one example, fifteen healthy volunteers were fitted with an apparatus of the invention. Modulated ultrasonic insonation was actuated according to autonomous instructions embedded in EEPROM memory of the device and continued while monitoring vascular and neurological status. Specific parameters for the ultrasonic wavepattern are generally as disclosed above. The insonation was continued for two hours without adverse effects. No adverse effects were reported in any of the tests.

According to embodiments of the invention, a headset 101 of the US device (see, for example, Fig. 1) is configured to permit a clinician to target critical vasculature without accompanying or preceding imaging studies, i.e., simply by fitting the headset onto the skull according to craniological landmarks that define a reference plane and the location of the major arteries. Accordingly, empirical studies were undertaken to determine what level of targeting of cerebral vasculature can be achieved with an embodiment of the invention. In such studies, transducer arrays of the headsets of the invention were modified to permit transcranial Doppler monitoring, where the "on-target" aim of the insonation was scored by detection of Doppler signals from the target vasculature. In one study, a review of case reports revealed that 86% of patients had detectable Doppler waveforms in the MCA and related cerebral vasculature. An average of 4.1 transducers received a return signal, indicating that multiple transducers were on target. In another study, MCA-localized Doppler was detected in 100% of all subjects; on average, 5.8 of the 12 temporally disposed transducers received Doppler return signals from the targeted MCA region and 2.7 of 4 transducers in the sub-occipital array received Doppler return signals from the targeted Basilar Artery. Combining the studies, 91% of the subjects showed evidence that the headset transducer arrays were correctly targeting the cerebral vasculature nexii of interest. The function of the headset to achieve rapid, unassisted, "passive" stereotactic targeting of the ultrasonic transducers onto key vascular targets is a factor in this success.

Improvements in 90 day outcomes were obtained by periodic follow-up treatment with ultrasound (without r-tPA) over a period of several days or weeks following an ischemic attack, and in fact the headset can be use prophylactically by itself if needed because of its capacity to non-invasively actuate endogenous mediators of thrombolysis.

The ultrasonic cerebral infarction therapeutic apparatus of this invention is an apparatus that was shown to dissolve thrombus responsible for cerebral infarction by irradiating the affected tissue with repeating cycle of ultrasonic wave patterns. The apparatus is useful for non-surgical application of ultrasound in ischemic stroke and, with superficial modification, for infarcted or embolic conditions of other vascular structures. In one embodiment, the apparatus functions autonomously without intervention, essentially as a pre-programmed automaton for delivering transcranial ultrasound, but optionally with sensors for collecting data and for adjusting operating parameters accordingly. In a first sensor mode, a transcranial ultrasound apparatus is configured with a phase detection circuit for verifying acoustic coupling before actuation of ultrasonic emissions. In another mode, a transcranial ultrasound apparatus is configured for adjusting $V_{BANG}$ using data on transducer voltage response. Using a multiplexed driving signal or signals, ultrasound is emitted by a plurality of transducers of an array while varying voltage to each individual transducer on the fly, thus improving reproducibility and consistency of insonation, which can be irregular due to variations in transducer manufacture.

Embodiments of the invention have been described as including a processor controlled by instructions stored in a memory. The memory may be random access memory (RAM), read-only memory (ROM), flash memory or any other memory, or combination thereof, suitable for storing control software or other instructions and data. Those skilled in the art should also readily appreciate that instructions or programs defining the functions of the present invention may be delivered to a processor in many forms, including, but not limited to, information permanently stored on non-writable storage media (e.g. read-only memory devices within a computer, such as ROM, or devices readable by a computer I/O attachment, such as CD-ROM or DVD disks), information alterably stored on writable storage media (e.g. floppy disks, removable flash memory and hard drives) or information conveyed to a computer through communication media, including wired or wireless computer networks. In addition, while the invention may be embodied in software, the functions necessary to implement the invention may optionally or alternatively be embodied in part or in whole using firmware and/or hardware components, such as combinatorial logic, Application Specific Integrated Circuits (ASICs), Field-Programmable Gate Arrays (FPGAs) or other hardware or some combination of hardware, software and/or firmware components.

While the invention is described through the above-described examples of embodiments, it will be understood by those of ordinary skill in the art that modifications to, and variations of, the illustrated embodiments may be made without departing from the inventive concepts disclosed herein. For example, the processor of an embodiment may be optionally pre-programmed to determine whether a degree of acoustic coupling of a given transducer of the headset to the subject's head is satisfactory, and to alert the clinician or other user if the quality of coupling should be improved. Craniological marks for positioning a headset may be selected from nasion, Lt otobasion superior (Lt OBS), Rt otobasion superior (Rt OBS), tragion, mandibular condyle, zygomatic arch, prosthion, or occipital prominence, while not limited thereto. At least three are selected to define a triangle and/or the reference plane. As a matter of convenient field use by untrained operators, the nasion/Lt OBS/Rt OBS triad has proven well suited. Mounting assemblies on the headset are configured with surfaces for engaging the landmarks of the head and stereotactically orienting the transducer arrays with respect to temporal and occipital acoustic windows into the cerebral arteries of said cranium so that the device may be used without further adjustment that would require an imaging modality such as transcranial Doppler, which is not readily available to first responders, for example. Furthermore, while some of the embodiments were described in reference to a remote control unit, it is appreciated that in a related embodiment at least some of the control-unit components (such as, for example, microprocessor, electronic circuitry, and/or storage medium) can be optionally incorporated into or built in or affixed to a headset assembly such as the headset assembly 101 of Fig. 1. In particular, it is within the scope of the invention to employ at least one of the headframe members 101a, 101b that is mechanically cooperated and in contact with a control-unit component.

Although some aspects of the invention have been described with reference to a flowchart, those skilled in the art should readily appreciate that functions, operations, decisions, etc. of all or a portion of each block, or a combination of blocks, of the flowchart may be combined, separated into separate operations or performed in other orders. Moreover, while the embodiments are described in connection with various illustrative data structures, one skilled in the art will recognize that the system may be embodied using a variety of data structures. In addition, the disclosed methods and structures may be used with appropriate alternative materials. Furthermore, disclosed aspects, or portions of these aspects, may be combined in ways not listed above. Accordingly, the invention should not be viewed as being limited to the disclosed embodiment(s).

## Claims

1.   An apparatus for transcranial sonothrombolysis of a subject's head, the apparatus comprising:

   a first temporal transducer array (105a) in cooperation with a first transducer-array registration member;
   a second temporal transducer array (105b) in cooperation with a second transducer-array registration member (154); and
   a headset (101) defining a headset plane (P, 230) and having a front corresponding to a forehead of the subject's head, a headset surface, and a nasion registration member (120) affixed to the headset (101) at the front and protruding transversely to the headset plane (P),,
   the first and second transducer arrays (105a, 105b) being affixed on said headset (101) opposite to one another, said nasion and first and second transducer-array registration members (120, 154) being configured such that, when the headset (101) is positioned on the subject's head for transcranial sonothrombolysis,

said nasion registration member (120) rests on the nasion (130) of the subject to ensure that the headset plane (P) is inclined with respect to a reference plane defined by a left otobasion superior (OBS), a right OBS, and a nasion (130) of the subject's head, and that the headset plane (P) passes through said left and right OBS, and said first and second transducer-array registration members (154) align said first and second temporal transducer arrays (105a, 105b) with a respective otobasion superior and a respective sphenoid shelf of the subject's head such as to optimize a degree of insonation of a target central cerebral vasculature with an ultrasound (US) beam emitted by said first and second transducer arrays (105a, 105b), the insonation being directed substantially along the headset plane (P).

2. An apparatus according to claim 1, wherein the headset plane (P) is inclined with respect to said reference plane by about 10 to about 15 degrees.

3. An apparatus according to claim 1, wherein the headset plane (P) is inclined with respect to said reference plane by about 12 degrees.

4. An apparatus according to claim 1, wherein said target central cerebral vasculature includes at least one of anterior cerebral artery (ACA), middle cerebral artery (MCA), posterior cerebral artery (PCA), internal carotid artery (ICA), basilar artery (BAS) and vertebral arteries (VER) and the Circle of Willis.

5. An apparatus according to claim 1, further comprising an occipital transducer array (106) affixed on said headset surface opposite to the nasion registration member (120) and configured to emit an ultrasound (US) beam substantially along the headset plane (P).

6. An apparatus according to claim 5, further comprising a hand-held portable remote control unit (102) configured to control an operation of at least one of said occipital and first temporal transducer arrays (106, 105a), and an umbilicus that operably connects said remote control unit (102) and said headset (101).

7. An apparatus according to claim 1, wherein said headset surface is a surface facing the subject's head when the headset (101) is positioned on the subject's head for transcranial sonothrombolysis.

8. An apparatus according to claim 1, wherein said headset (101) includes anterior and posterior headframe members (101a, 101b) configured to mate at corresponding ends of said members (101a, 101b) such as to define said headset plane (P) and a circumcranial loop of said headset (101).

9. An apparatus according to claim 8, wherein mutual positioning of the anterior and posterior headframe members (101a, 101b) is tensionably adjustable.

10. An apparatus according to claim 1, further comprising:

   a processor (115);
   a non-transitory tangible computer-readable medium; and
   computer-readable program code encoded in said computer-readable medium, the computer-readable program code comprising series of computer readable program steps to effect:

      insonating, in the headset plane (P), the target central cerebral vasculature; and
      defining a regime of operation of the at least one of said transducer arrays (105a, 105b).

11. An apparatus according to claim 10, wherein said computer-readable program code comprises series of computer readable program steps to effect such insonating of the target central cerebral vasculature that a peak rarefaction pressure associated with the insonation does not exceed 300 kPa and a thermal index does not exceed a physiologically compatible thermal index at a predetermined depth within the subject's head.

12. An apparatus according to claim 1, wherein registration members of the apparatus are positioned such as to ensure that, when the headset is positioned on the subject's head for transcranial sonothrombolysis, transducer arrays are passively located, without a need for re-iterative reorientation of the transducer arrays and without a need for any feedback information characterizing the precision of such positioning, in such a spatial relationship with external craniological marks as to emit ultrasound towards cerebral arteries most commonly associated with stroke.

**Patentansprüche**

1. Eine Vorrichtung zur transkranialen Sonothrombolyse eines Probandenkopfes, die Vorrichtung umfassend:

   eine erste zeitliche Wandleranordnung (105a) in Kooperation mit einem ersten Wandleranordnungs-Registierungsglied;
   eine zweite zeitliche Wandleranordnung (105b) in Kooperation mit einem zweiten Wandleranordnungs-Registierungsglied (154); und
   einem Headset (101), welches eine Heatset-Ebene (P, 230) definiert
   und welches eine Front hat,
   die zu einer Stirn des Probandenkopfes korrespondiert, eine Headset-Oberfläche beziehungsweise Headset-Fläche und ein Nasenwurzel(engl. Nasion)-Registrierungsglied (120) befestigt an dem Headset (101) an der Front und transversal hervorstehend zu der Headset-Ebene (P),
   wobei die ersten und zweiten Wandleranordnungen (105a, 105b) an dem Headset (101) gegenüberliegend zueinander befestigt sind,
   wobei die Nasenwurzel- und erste und zweite Wandleranordnungs-Registierungsglieder (120, 154) derart konfiguriert sind, dass wenn das Headset (101) auf dem Probandenkopf zur transkranialen Sonothrombolyse positioniert ist,
   das Nasenwurzel-Registrierungsglied (120) auf der Nasenwurzel (130) des Probanden aufliegt um sicherzustellen, dass die Headset-Ebene (P) geneigt ist im Bezug auf eine Referenzebene, welche durch einen linken oberen Ohrmuschelansatz (engl. Otobasion superior (OBS)), einen rechten OBS und eine Nasenwurzel (130) des Probandenkopfes definiert ist, und das die Headset-Ebene (P) durch die linke und rechte OBS verläuft, und die ersten und zweiten Wandleranordnungs-Registierungsglieder (154) die ersten und zweiten Wandleranordnungen (105a, 105b) mit einem jeweiligen oberen Ohrmuschelansatz und einem jeweiligen Keilbein-Boden (engl. sphenoid shelf) des Probandenkopfes auf eine Weise ausrichten, um einen Grad von Ultraschallaussetzung (engl. insonation) eines Ziels zentraler zerebaler Blutgefäße mit einem Ultraschallstrahl (US) zu optimieren, welcher von den ersten und zweiten Wandleranordnungen (105a, 105b) emittiert wird, wobei die Ultraschallaussetzung im Wesentlichen entlang der Headset-Ebene (P) gerichtet ist.

2. Eine Vorrichtung gemäß Anspruch 1, wobei die Headset-Ebene (P) im Bezug auf die Referenzebene um etwa 10 bis etwa 15 Grad geneigt ist.

3. Eine Vorrichtung gemäß Anspruch 1, wobei die Headset-Ebene (P) im Bezug auf die Referenzebene um etwa 12 Grad geneigt ist.

4. Eine Vorrichtung gemäß Anspruch 1, wobei das Ziel zentraler zerebraler Blutgefäße zumindest eine der vorderen zerebralen Arterie (ACA), mittleren zerebralen Arterie (MCA), hinteren zerebralen Arterie (PCA), internen Halsschlagader (ICA), Basilararterie (BAS) und Wirbelarterien (VER) und den Arterienring des Gehirns (engl. Circle of Willis) beinhaltet.

5. Eine Vorrichtung gemäß Anspruch 1, weiter umfassend eine Hinterkopf-Wandleranordnung (106) befestigt an der Headset-Fläche gegenüberliegend zu dem Nasenwurzel-Registrierungsglied (120) und konfiguriert um einen Ultraschallstrahl (US) im Wesentlichen entlang der Headset-Ebene (P) zu emittieren.

6. Eine Vorrichtung gemäß Anspruch 5, weiter umfassend ein tragbares Handfernbedienungsgerät (102), welches konfiguriert ist um einen Betrieb von zumindest einen der Hinterkopf- und ersten zeitlichen Wandleranordnungen (106, 105a) zu bedienen und einen Nabel, der das Fernbedienungsgerät (102) und das Headset (101) betriebsfähig verbindet.

7. Eine Vorrichtung gemäß Anspruch 1, wobei die Headset-Fläche eine Fläche zugewandt dem Probandenkopf ist, wenn das Headset (101) auf dem Probandenkopf zur transkranialen Sonothrombolyse positioniert ist.

8. Eine Vorrichtung gemäß Anspruch 1, wobei das Headset (101) vordere und hintere Kopfgestellglieder (101 a, 101 b) beinhaltet, welche konfiguriert sind um an korrespondierenden Enden der Glieder (101 a, 101 b) derart zusammenzupassen, um die Headset-Ebene (P) und eine kopfumfassende Schleife des Headset (101) zu definieren.

9. Eine Vorrichtung gemäß Anspruch 8, wobei gegenseitiges Positionieren der vorderen und hinteren Kopfgestellglieder (101 a, 101 b) spannbar einstellbar ist.

**10.** Eine Vorrichtung gemäß Anspruch 1, weiter umfassend:

einen Prozessor (115);
ein nicht transitorisches, greifbares, computerlesbares Medium; und
computerlesbarer Programmcode codiert in dem computerlesbaren Medium, wobei der computerlesbare Programmcode Serien von computerlesbaren Programmschritten umfasst um zu bewirken:

Ultraschallaussetzen eines Ziels zentraler zerebraler Blutgefäße in der Headset-Ebene (P); und
Definieren einer Ordnung des Arbeitsablaufes von den zumindest einen der Wandleranordnungen (105a, 105b).

**11.** Eine Vorrichtung gemäß Anspruch 10, wobei der computerlesbare Programmcode Serien von computerlesbaren Programmschritten umfasst um solch Ultraschallaussetzen des Ziels zentraler zerebraler Blutgefäße derart zu bewirken, dass ein Verdünnungsdruckhöchstwert verknüpft mit der Ultraschallaussetzung 300 kPA nicht überschreitet und ein thermischer Index einen physiologisch verträglichen thermischen Index in einer vorgegebenen Tiefe innerhalb des Probandenkopfes nicht überschreitet.

**12.** Eine Vorrichtung gemäß Anspruch 1, wobei wenn das Headset auf dem Probandenkopf zur transkranialen Sonothrombolyse positioniert ist, Registrierungsglieder der Vorrichtung derart positioniert sind um sicherzustellen, dass Wandleranordnungen in solch einer räumlichen Beziehung mit externen kraniologischen Markierungen passiv lokalisiert werden, ohne einer Notwendigkeit zur sich wiederholenden Neuausrichtung der Wandleranordnungen und ohne einer Notwendigkeit für jegliche Rückmeldungsinformation, welche die Genauigkeit einer solchen Positionierung charakterisiert, um Ultraschall in Richtung zerebraler Arterien, die am häufigsten in Verbindung stehen mit Schlagfall, zum emittieren.

**Revendications**

**1.** Appareil de sonothrombolyse transcrânienne de la tête d'un sujet, l'appareil comprenant :

un premier réseau de transducteurs temporaux (105a) en coopération avec un premier élément d'enregistrement de réseau de transducteurs ;
un second réseau de transducteurs temporaux (105b) en coopération avec un second élément d'enregistrement de réseau de transducteurs (154) ; et
un casque (101) définissant un plan de casque (P, 230) et ayant une partie avant correspondant à un front de la tête du sujet, une surface de casque et un élément d'enregistrement de nasion (120) fixé au casque (101) à l'avant et faisant saillie de manière transversale au plan de casque (P),
les premier et second réseaux de transducteurs (105a, 105b) étant fixés sur ledit casque (101) à l'opposé l'un de l'autre,
lesdits éléments d'enregistrement de nasion et de premier et second réseaux de transducteurs (120, 154) étant configurés de sorte que, lorsque le casque (101) est positionné sur la tête du sujet pour une sonothrombolyse transcrânienne,
ledit élément d'enregistrement de nasion (120) repose sur le nasion (130) du sujet pour garantir que le plan de casque (P) est incliné par rapport à un plan de référence défini par un otobasion supérieur gauche (OBS), un OBS droit et un nasion (130) de la tête du sujet et que le plan de casque (P) passe à travers lesdits OBS gauche et droit, et
lesdits éléments d'enregistrement de premier et second réseaux de transducteurs (154) alignent lesdits premier et second réseaux de transducteurs temporaux (105a, 105b) avec un otobasion supérieur respectif et un étage sphénoïde respectif de la tête du sujet de manière à optimiser un degré d'insonation d'une vasculature cérébrale centrale cible avec un faisceau d'ultrasons (US) émis par lesdits premier et second réseaux de transducteurs (105a, 105b), l'insonation étant dirigée essentiellement le long du plan de casque (P).

**2.** Appareil selon la revendication 1, dans lequel le plan de casque (P) est incliné par rapport audit plan de référence d'environ 10 à environ 15 degrés.

**3.** Appareil selon la revendication 1, dans lequel le plan de casque (P) est incliné par rapport audit plan de référence d'environ 12 degrés.

**4.** Appareil selon la revendication 1, dans lequel ladite vasculature cérébrale centrale cible inclut au moins une artère parmi l'artère cérébrale antérieure (ACA), l'artère cérébrale centrale (MCA), l'artère cérébrale postérieure (PCA), l'artère carotidienne interne (ICA), l'artère basilaire (BAS) et des artères vertébrales (VER) et le cercle artériel de Willys.

**5.** Appareil selon la revendication 1, comprenant en outre un réseau de transducteurs occipitaux (106) fixés sur ladite surface de casque opposée à l'élément d'enregistrement de nasion (120) et configurés pour émettre un faisceau d'ultrasons (US) essentiellement le long du plan de casque (P).

**6.** Appareil selon la revendication 5, comprenant en outre un module de commande à distance portable mobile (102) configuré pour commander une opération d'au moins un desdits réseau de transducteurs occipitaux et premier réseau de transducteurs temporaux (106, 105a), et un ombilic qui connecte de manière opérationnelle ledit module de commande à distance (102) et ledit casque (101).

**7.** Appareil selon la revendication 1, dans lequel ladite surface de casque est une surface tournée vers la tête du sujet lorsque le casque (101) est positionné sur la tête du sujet pour une sonothrombolyse transcrânienne.

**8.** Appareil selon la revendication 1, dans lequel ledit casque (101) inclut des éléments de montant de tête antérieur et postérieur (101a, 101b) configurés pour se conjuguer à des extrémités correspondantes desdites éléments (101a, 101b) de manière à définir ledit plan de casque (P) et une boucle péricrânienne dudit casque (101).

**9.** Appareil selon la revendication 8, dans lequel un positionnement mutuel des éléments de montant de tête antérieur et postérieur (101a, 101b) est ajustable par tension.

**10.** Appareil selon la revendication 1, comprenant en outre :

un processeur (115) ;
un support lisible par ordinateur tangible non transitoire ; et
du code de programme lisible par ordinateur dans ledit support lisible par ordinateur, le code de programme lisible par ordinateur comprenant une série d'étapes de programme lisible par ordinateur pour effectuer :

l'insonation dans le plan de casque (P) de la vasculature cérébrale centrale cible ; et
la définition d'un régime de fonctionnement de l'au moins un desdits réseaux de transducteurs (105a, 105b).

**11.** Appareil selon la revendication 10, dans lequel ledit code de programme lisible par ordinateur comprend une série d'étapes de programme lisible par ordinateur pour effectuer une insonation de la vasculature cérébrale centrale cible telle qu'une pression de raréfaction maximale associée à l'insonation ne dépasse pas 300 kPa et un indice thermique ne dépasse pas un indice thermique compatible du point de vue physiologique à une profondeur prédéterminée au sein de la tête du sujet.

**12.** Appareil selon la revendication 1, dans lequel des éléments d'enregistrement de l'appareil sont positionnés de manière à garantir que, lorsque le casque est positionné sur la tête du sujet pour une sonothrombolyse transcrânienne, des réseaux de transducteurs sont localisés de manière passive, sans besoin de réorientation réitérative des réseaux de transducteurs et sans besoin de toute information de rétrocontrôle caractérisant la précision d'un tel positionnement, dans un rapport spatial avec des marques craniologiques externes tel qu'ils émettent des ultrasons vers des artères cérébrales les plus couramment associées à un accident vasculaire cérébral.

## Fig. 1

*Fig.*2A

*Fig.*2B

*Fig.* 4A

*Fig.* 4B

*Fig.* 4C

**Fig. 3**

**Fig. 5A**

**Fig. 5B**

**Fig. 6**

**Fig. 7A**

**Fig. 7B**

**Fig. 7C**

Fig. 7D

**Fig. 8A**

**Fig. 8B**

*Fig. 9A*

*Fig. 9B*

**Fig. 9C**

### Fig. 11A

### Fig. 11B

*Fig. 12A*

344

TDX

343a

RT1

LT1

OC1

RT2

LT2

OC2

RT3

LT3

OC3

RT4

LT4

OC4

RT5

LT5

RT6

LT6

343n

## Fig. 12B

<u>346</u>

## Fig. 12C

<u>348</u>

*Fig. 13*

*Fig.* 14A

*Fig.* 14B

*Fig.* 15A

*Fig.* 15B

FIG. 16A

FIG. 16B

*Fig.* 17

## FOR HANDS-FREE OPERATION WITH AUTOMATED COUPLING SENSOR

390

```
┌─────────────────────────────┐
│      ATTACH HEADSET         │  ⎰391
│   TO REMOTE CONTROLLER      │
└─────────────────────────────┘
              │
              ▼
┌─────────────────────────────┐
│   USING REMOTE CONTROLLER,  │  ⎰392
│      CYCLE POWER ON;        │
└─────────────────────────────┘
              │
              ▼
┌─────────────────────────────┐
│     APPARATUS VERIFIES      │
│     ACOUSTIC COUPLING       │  ⎰393
│    OF EACH TRANSDUCER;      │
└─────────────────────────────┘
              │
              ▼
┌─────────────────────────────┐
│    IF ALL SYSTEMS ARE GO,   │
│    EMISSIONS WILL BEGIN;    │  ⎰394
└─────────────────────────────┘
              │
              ▼
┌─────────────────────────────┐
│   AFTER DEFINED DURATION OF │
│  PATTERNED ULTRASONIC EMISSIONS,  │  ⎰395
│   APPARATUS WILL POWER DOWN;│
└─────────────────────────────┘
              │
              ▼
┌─────────────────────────────┐
│      REPEAT POWER CYCLE     │  ⎰396
│        IF DESIRED.          │
└─────────────────────────────┘
```

*Fig.* 18A

$$Z_{mag} = \sqrt{R^2 + L_C^2}$$

*Fig.* 18B

FIG. 19

*Fig.* 20A

*Fig.* 20B

**REFERENCES CITED IN THE DESCRIPTION**

**Patent documents cited in the description**

- US 2007167765 A1

- US 3961140 A

**Non-patent literature cited in the description**

- **HENRIKSON.** CT evaluation of plastic intraocular foreign bodies. *Am. J. Neuroradiology,* 1987, vol. 8, 378-79

- **CINTAS et al.** High Rate of Recanalization of Middle Cerebral Artery Occlusion During 2-MHz Transcranial Color-Coded Doppler Continuous Monitoring Without Thrombolytic Drug. *Stroke,* 2002, vol. 33, 626-628